# EUROPEAN PATENT SPECIFICATION

(11) **EP 1 507 863 B1**
(45) Date of publication and mention of the grant of the patent: **27.12.2006**
(21) Application number: 03752862.7
(22) Date of filing: 19.05.2003
(51) Int. Cl.: C12N 15/68, C12N 15/74, C12N 15/64, A61K 39/112, A61K 39/02, A61K 35/74, A61K 48/00, C12N 1/36, C12R 1/42

(54) **PLASMID STABILISATION IN VIVO**
IN VIVO STABILSIERUNG VON PLASMIDEN
STABILISATION DE PLASMIDE IN VIVO

(30) Priority: 18.05.2002 GB 0211459
(43) Date of publication of application: 23.02.2005
(73) Proprietor: Cobra Biologics Limited, Keele, Newcastle Staffordshire ST5 5SP (GB)
(72) Inventor: HANAK, Julian, Cobra Therapeutics Limited, Keele ST5 5SP (GB); CRANENBURGH, Rocky, Cobra Therapeutics Limited, Keele ST5 5SP (GB); GORMAN, Scott, Pharmaventures Limited, Oxford OX4 4GA (GB)
(74) Representative: Hallybone, Huw George
(86) International application number: PCT/GB2003/002166
(87) International publication number: WO 2003/097838

(56) References cited:
- WO-A-01/83785
- WO-A-97/09435
- QIAN FENG ET AL: "Construction of a tetR-integrated Salmonella enterica serovar Typhi CVD908 strain that tightly controls expression of the major merozoite surface protein of Plasmodium falciparum for applications in human vaccine production." INFECTION AND IMMUNITY, vol. 70, no. 4, April 2002 (2002-04), pages 2029-2038, XP002253524 ISSN: 0019-9567
- CRANENBURGH R M ET AL: "Escherichia coli strains that allow antibiotic-free plasmid selection and maintenance by repressor titration." NUCLEIC ACIDS RESEARCH. ENGLAND 1 MAR 2001, vol. 29, no. 5, 1 March 2001 (2001-03-01), page E26 XP002253525 ISSN: 1362-4962 cited in the application
- WILLIAMS STEVEN G ET AL: "Repressor titration: A novel system for selection and stable maintenance of recombinant plasmids." NUCLEIC ACIDS RESEARCH, vol. 26, no. 9, 1 May 1998 (1998-05-01), pages 2120-2124, XP002253526 ISSN: 0305-1048
- GALEN JAMES E ET AL: "Optimization of plasmid maintenance in the attenuated live vector vaccine strain Salmonella typhi CVD 908-htrA." INFECTION AND IMMUNITY, vol. 67, no. 12, December 1999 (1999-12), pages 6424-6433, XP002253527 ISSN: 0019-9567
- TITBALL RICHARD W ET AL: "Expression of the Yersinia pestis capsular antigen (F1 antigen) on the surface of an aroA mutant of Salmonella typhimurium induces high levels of protection against plague" INFECTION AND IMMUNITY, AMERICAN SOCIETY FOR MICROBIOLOGY. WASHINGTON, US, vol. 65, no. 5, 1997, pages 1926-1930, XP002164415 ISSN: 0019-9567 cited in the application
- CÁRDENAS L ET AL: "Stability, immunogenicity and expression of foreign antigens in bacterial vaccine vectors." VACCINE. ENGLAND 1993, vol. 11, no. 2, 1993, pages 126-135, XP002253528 ISSN: 0264-410X
- MASTROENI P ET AL: "Salmonella: Immune responses and vaccines." VETERINARY JOURNAL, vol. 161, no. 2, March 2001 (2001-03), pages 132-164, XP008021618 ISSN: 1090-0233
- GARMORY HELEN S, ET AL: "Antibiotic-free plasmid stabilization by operator-repressor titration for vaccine delivery by using live Salmonella entetica serovar Typhimurium" INFECTION AND IMMUNITY, vol. 73, no. 4, April 2005 (2005-04), pages 2005-2011, XP008052479

## Description

### FIELD OF THE INVENTION

This invention relates to a transformed attenuated bacterial cell for use as a medicament.

### BACKGROUND TO THE INVENTION

The expression of recombinant proteins in host cells is important for a number of therapeutic applications. In particular, expression of antigenic proteins in a suitable host cell is an efficient method of immunisation against pathogenic organisms. A convenient method of achieving this is to generate a recombinant host cell containing a plasmid that encodes one or more antigens or epitopes. However, in order to maintain such plasmids at a sufficient copy number, some selective pressure is required. This is because extrachromosomal DNA carried in host cells is inherently unstable since cells that contain plasmids usually have an increased metabolic burden compared to plasmid-free segregant cells.

The conventional method of maintaining copy number of desired plasmids in cells in culture is to include a selectable marker, such as an antibiotic resistance gene, on the plasmid, and to culture the cells in the presence of the appropriate antibiotic. For cells or plasmids intended for therapeutic use, this has certain disadvantages even in *in vitro* culture, for instance the potential risk of spread of antibiotic resistance genes. For example, for *in vivo* use, the required presence of a sufficient concentration of antibiotic to ensure selection of cells containing a high copy number of plasmids may be undesirable, impractical, or impossible.

Other successful methods for maintaining plasmids do not utilise antibiotic selection but rather rely on a mutant host which is unable to synthesise an essential metabolite and inserting the gene which provides for this synthesis in the plasmid. Other solutions involve placing a gene coding for a toxic product in the chromosome and then including a corresponding inhibitor or repressor system in the plasmid, with the result that plasmid-free cells are effectively killed upon segregation.

Qian Feng et al (Infection and Immunity, 2002, 70:2029-2038) and Mastroeni et al (Veterinary Journal, 2001, 161(2):132-164) disclose methods of expressing a gene of interest in a transformed cell within a recipient organism EP 0851932 describes a method of maintaining plasmids within host cells in *in vitro* culture by means of operator repressor titration. This involves engineering a suitable host cell, such that expression of an essential chromosomal gene is rendered conditional by the insertion of an operator sequence into its control region. This operator binds a repressor protein, and in the presence of such bound repressor, expression of the gene is downregulated and, in the absence of its essential product, the cell dies or fails to divide. The chromosome of the host cell is further engineered so as to contain a gene expressing the repressor protein, so that the cell is not now viable unless the gene is depressed in some way or the cell's auxotrophy is rescued by addition of the required essential product as a supplemental nutrient in the culture medium.

The desired plasmids for maintenance in this host cell are engineered to contain one or more operator sites similar to the one controlling expression of the essential host cell gene as described above. When transformed into the host cell, such plasmids compete for binding of the repressor within the cell. If a sufficient copy number is reached, all the repressor is bound by the plasmids and the essential cellular gene is derepressed, releasing the cell from its auxotrophy and allowing it to grow and divide in the absence of supplemental nutrients.

Although EP 0851932 (and Cranenburgh et al, 2000, Nucleic Acids Research 29:E26) describes such a system for use *in vitro,* there is no disclosure of suitable host cells and plasmids for safe and convenient *in vivo* therapeutic use. Consequently, there is a need for such a system for the convenient expression of a variety of plasmid-borne therapeutic genes in acceptable host cells for *in vivo* therapeutic use.

### SUMMARY OF THE INVENTION

The invention is based on modified host cells, for *in vivo* therapeutic use, that may be used to express a therapeutically useful plasmid-borne gene, using a plasmid maintenance system that does not require the use of a plasmid-borne dominant selectable marker, but rather utilises a system of repressor titration.

One particularly useful application of this is in the field of vaccination. The earliest forms of prophylactic immunisation involved use of attenuated strains of pathogenic organisms, which carried antigens shared with the more dangerous wild type organisms. The immune reaction generated against the attenuated strains was protective against infection with the wild type organisms. This approach is still used today, for instance in the use of the BCG strain of *Mycobacterium bovis.*

As used herein, "immunisation" means the stimulation of protective or therapeutic immune response. "Vaccination" is the deliberate administration of antigens capable of stimulating a protective or therapeutic immune response for the purpose of prophylaxis. In this context such antigens may be referred to as "immunogens".

An "attenuated" organism, in this context, is one which elicits a reduced pathogenic effect (ideally little or none) when administered to a recipient organism, as compared to a wild-type pathogen. Such attenuated organisms may arise naturally, may be deliberately selected for by repeated passage *in vivo* or *in vitro,* or may be constructed by recombinant DNA technology by manipulating or removing genes known to be associated with virulence.

However, there are limitations to the approach of using attenuated strains of particular pathogens as vaccines against the infection caused by their wild-type equivalents. No safe attenuated strain of the relevant pathogen may be available, or such a strain may generate a poorly protective immune response. The use of recombinant DNA techniques allows, in principle, the identification of genes responsible for the expression of specific antigens by pathogens, which are capable of generating a protective immune response, and transfer of them to safer, or more convenient, host cells that may then be used as vaccines. Such genes may directly encode protein antigens, or may encode enzymes responsible for the biosynthesis of more complex carbohydrate or lipid antigenic structures. Moreover, this allows the use of a limited number of attenuated host strains to deliver recombinant antigens from any of a wide range species of pathogens.

Antigen-associated genes transferred to a more convenient and safer (preferably attenuated) host cell in this way may, in principle, either be inserted (integrated) into the host chromosome, or carried on an extrachromosomal clement such as a self-replicating plasmid. The advantage of integration is that the gene is stably maintained as part of the chromosome. The disadvantage is that such stable integration is a complex and time-consuming procedure, particularly where a number of different antigen-associated genes are to be expressed. Random integration may produce unexpected effects due to insertional inactivation of host cell genes. Site-specific integration by homologous recombination is complex and requires considerable effort and care.

Vaccination can be carried out with recombinant attenuated organisms that express appropriate antigen-associated genes carried on extrachromosomal elements such as plasmids (for example, Oyston et al, 1995, Infect. Immun. 63:563-568; Titball et al, 1995, Infect. Immun. 63: 563-568). Construction of suitable expression vectors is comparatively simple, and transformation or transfection with such vectors is routine for many types of cell. The disadvantage is that such plasmids are not stably replicated over repeated cell divisions in the absence of some means of selection. Conventionally, in the case of transformed cells in culture, this is achieved by means of a dominant selectable marker, almost always an antibiotic resistance gene, carried on the plasmid. The cells grow in a medium to which the relevant antibiotic is added at an appropriate concentration and only cells carrying the plasmid, and hence expressing the resistance gene, survive and divide. This clearly has major practical difficulties when applied to the *in vivo* use of such cells for therapeutic use, such as vaccination. In addition to the risk of spreading antibiotic resistance and the possibility of an inappropriate immune response to the product of the resistance gene, it may be difficult or impossible to maintain a sufficient concentration of antibiotic in the appropriate tissue or compartment of the recipient organism. It is, in any case, undesirable to expose the recipient to the required dose of antibiotic if this can be avoided.

The current invention allows stable maintenance of a plasmid within a host cell, in the absence of any external selection such as an antibiotic. Operator repressor titration allows plasmids to be self-selecting in a suitably modified host cell. Such a modified host cell ('ORT^{®} host cell') needs to be engineered in respect to two chromosomal genes, but once constructed, may be used for any number of 'ORT^{®} plasmids' expressing different transgenes. The ability to express any number of different antigens using the same attenuated (and validated) host cell, simply by transforming with an ORT® plasmid encoding the appropriate antigen-associated gene, would be a great advantage. The lack of a requirement for any added external selection pressure allows host cells placed within a recipient organism to stably maintain plasmids encoding and expressing a useful therapeutic gene (such as an immunogenic antigen or epitope therefrom).

Equally importantly, the invention allows transformation of a plasmid free of any antibiotic resistance gene into a suitably modified bacterial (or other) host cell, with subsequent preferential growth and selection of resulting transformants, in the absence of antibiotics.

The current invention is, therefore, particularly useful for the expression of one or more antigenic proteins or peptides, or of enzymes that are required for the biosynthesis of non-peptide antigens, in host cells, in order to administer such recombinant host cells to a recipient organism for the purpose of generating a protective immune response against the pathogen from which said antigen(s) was derived. Such host cells may or may not continue to grow and divide in said recipient organism. The plasmid-borne gene(s) encoding the antigen(s) may be expressed in the host cell prior to administration to the recipient organism or only after administration to the recipient organism. The stage at which the antigen is expressed may be regulated by placing the plasmid-borne gene under the control of different promoters. For example, where the host cell is a bacterial cell and the plasmid-borne gene is under the control of a bacterial promoter, the antigen will be expressed in host cells in which the plasmid is maintained prior to administration to a recipient organism. Where the host cell is a bacterial cell and the plasmid-borne gene is under the control of a eukaryotic promoter, the antigen will not be expressed in host cells prior to administration but will be expressed following administration of host cells which have maintained the plasmid to a eukaryotic recipient organism, as shown in Galmory *et al* (FEMS Microbiol Rev., 2002, 26(4):339-53).

The skilled person will be aware of antigens that can be used to generate an immune response against pathogens and the plasmid administered to the host cell of the invention may encode any of these antigens. For example, the *caf* operon from *Y. pestis* has been shown to be a promising vaccine candidate inducing high levels of immunity against *Y. pestis* (Titball *et al,* 1997, Infection & Immunity, 65: 1926-1930). An ORT host cell comprising an ORT plasmid comprising the caf operon could therefore be used to generate an immune response against *Y. pestis.*

Alternatively, the antigens may be derived from a tumour, and the technique used to generate an anti-tumour immune response with the aim of destroying the tumour, or at least of slowing its growth.

In an alternative embodiment, the invention may be used to express and deliver therapeutic gene products for purposes other than generating an immune response. In principle, a wide range ofrecombinant therapeutics might be delivered by this method.

For instance, proteins may be delivered to replace the missing or reduced product of a defective gene, such as a-1-antitrypsin in a-1-antitrypsin deficiency, or Factor VIII in haemophilia. Particularly suitable would be applications where therapeutics are secreted into the lumen of the gut by introduced recombinant organisms produced according to the invention. Examples include the production of pancreatic enzymes to replace natural enzymes reduced or absent as a result of cystic fibrosis, pancreatitis or pancreatectomy, or production of extra vitamin K.

It is envisaged that the product of the gene encoded on the ORT® plasmid might be expressed on the surface of the cell (particularly useful for vaccination purposes) or secreted from the host cell. In the case of a bacterial host cell capable of surviving within the cells of the recipient organism (for example Listeria and Salmonella) this system allows recombinant antigens to be introduced directly into intracellular compartments, which may have advantages in the case of antigen presenting cells, influencing the type of subsequent presentation by molecules of the major histocompatibility complex. Such advantages will be clear to one of skill in the appropriate art. Suitable methods for secretion of antigens expressed in gram-negative bacteria include the haemolysin secretion system of *E coli* (Gentschev ct al, 1996, Gene 179; 133; Gentschev et al, 2001, Vaccine 79:2621).

The invention may also be used to deliver therapeutic gene products which are the transcription products of the plasmid-borne genes, such as antisense oligonucleotides. It is also envisaged that the invention may be used to deliver plasmid-borne genes which are themselves therapeutic products, for example for gene therapy purposes.

The invention encompasses a transformed attenuated bacterial (host cell containing a plasmid comprising a gene of interest and an operator susceptible to binding by a repressor expressed in *trans,* a second gene encoding the repressor present on a chromosome, and a third chromosomal gene that is functionally associated with an operator and essential for cell growth, wherein the plasmid is present in the cell in sufficient numbers to titrate the repressor such that the essential gene is expressed, thereby permitting cell growth.

Preferably, the third gene is not an antibiotic resistance gene.

As used herein, "functionally associated" or "operatively associated", with respect to any operator sequence and an associated gene, means that the operator is linked in *cis* to the gene such that expression of the gene is susceptible to repression upon binding of a repressor to the operator.

It will be understood by one of skill in the art that the term operator is used to define any nucleic acid sequence to which a repressor binds to prevent transcription from an associated promoter. The operator sequence present on the plasmid need not be a sequence that is identical to the operator sequence on the chromosomal gene, in that the plasmid operator need only consist of the minimal sequences necessary for binding the repressor that represses transcription of the chromosomal gene. It will also be understood that mutated operator sequences are also useful according to the invention, for example, sequences having one or more nucleotides inserted, deleted, or substituted which result in increased or decreased affinity for the corresponding repressor.

As used herein, "cell growth" refers to increasing numbers of cells in a culture medium or *in vivo* over time, and also refers to cell survival where the number of cells does not increase over time, but rather the number of live cells does not decrease over time.

Preferably, the repressor gene encodes one of the *E. coli* lac repressors, in particular the lac I^{q} repressor, the *E coli* trp repressor, the *E. coli* galR repressor, the *E. coli* araC repressor, the *E. coli* ArgRNV repressor (Burke et al (1994) Mol. Microbiol., 13, 609-618) and the *E.coli* Tet repressor.

As described above, each repressor is operative in *trans* with a *trans*-associated operator sequence that is present both in the chromosome and on the plasmid. The invention contemplates the presence of one or more repressor genes on the host chromosome, e.g., one, two or three repressor genes, in order to ensure plasmid stability where one chromosomal repressor gene becomes mutated or deleted.

Preferred operator sequences therefore include the lac operator, the A operator, the trp operator, the gal operator, the ara operator, the Arg operator and the Tet operator. If desired, the corresponding promoter may be functionally associated with its operator.

More than one different essential chromosomal gene that is functionally associated with the operator may be present in the cell chromosome, wherein the essential gene is linked to an operator and therefore susceptible to repression by the repressor, to guard against loss of repressor susceptibility at one chromosomal operator. In one preferred embodiment of the invention, the gene encoding the repressor protein is present in two or three copies at different locations in the chromosome to guard against loss of repressor expression at one chromosomal location.

Preferred essential genes that are located on the host chromosome include but are not limited to genes falling within the following categories: genes encoding products related to the biosynthesis of cell metabolites such as cell wall precursors, genes whose products are involved in carbon metabolism, genes coding for antibiotic resistance, and genes encoding biosynthesis or regulation of macromolecules, e.g., genes essential for DNA and/or RNA synthesis and replication functions.

Preferably, the plasmid comprises an origin of replication permitting replication of about 20 copies of the plasmid per host cell, or as much as about 200 copies of the plasmid per host cell. Examples of such plasmids include but are not limited to those containing the pBR322 origin of replication and the pUC series of plasmids as described by Vieira & Messing (1982, Gene, 19(3), 259268) and Yanisch-Perron et al. (1985, Gene, 33(1), 10S-119), herein referred to as pUC. Alternative plasmids that may be used include the pAHL plasmid, derived from the pAH34L plasmid described by Titball et al (1997, Infection & Immunity, 65: 1926-1930).

As used herein, "origin of replication" refers to those sequences on the plasmid that are necessary for maintaining the plasmid at a given copy number per cell.

The repressor titration system described herein enables the stable maintenance of plasmids in moderate or high copy number without the use of plasmid-encoded dominant selectable markers, such as for antibiotic resistance, and can be used with any host that can support a trans-acting repressor/operator system. One advantage of the invention is in its reliance on plasmid maintenance other than by antibiotic selection of plasmid-bearing cells. The absence of dominant selectable markers, such as antibiotic resistance genes, on the plasmid, as described herein, is also advantageous in that it avoids the potentially serious problems related to expression of those genes in a recipient organism, especially a human and that it avoids wide-spread use of bacterial genes encoding antibiotic resistance, which use tends to promote transfer of such genes in the bacterial population as a whole.

The transformed attenuated bacterial cell, is preferably an enterobacterial cell, further preferably a cell of the genus *Salmonella,* and most preferably a cell of the species *Salmonella typhimurium* or of the species *Salmonella typhi.*

It is further preferred that said transformed attenuated bacterial cell has been attenuated to reduce its pathogenicity by a modification of its *aroA* gene, *aroC* gene, *aroD* gene or *htrA* gene. The transformed cell may contain a modification of more than one of these genes.

Most preferably, the attenuated transformed cell is of the strain *Salmonella typhimurium* SL3261 or of the strain *Salmonella typhi* CVD 908-htr.

It is also preferred that said third gene contained by the cell encodes an enzyme required for bacterial cell wall synthesis. More preferably said gene is *dapD.*

Alternatively, said third gene contained by the cell encodes an enzyme required for fatty acid synthesis. More preferably, said third gene is *fabA.*

The gene of interest may be any gene which it is desired to deliver to the recipient organism. The gene of interest may encode an antigen or the transcription product of the gene of interest may be an antisense oligonucleotide. Preferably, the gene of interest is functionally associated with a bacterial promoter or a eukaryotic promoter.

The transformed attenuated bacterial cell is used as a medicament. The use of the transformed cell as a medicament will depend on the nature of the gene of interest. Preferably, the transformed cell may be used as a gene delivery system. For example, the transformed cell may be used to deliver a gene of interest for gene therapy purposes. The transformed cell may also be used to deliver a gene of interest, the transcription product of which has a therapeutic effect, such as an antisense oligonucleotide or to deliver a gene of interest which encodes an antigen which has a therapeutic effect.

Alternatively, the transformed cell may be used as an immunogen. According to this embodiment, the gene of interest may encode one or more antigens which elicit an immune response in the recipient organism. The skilled person will be aware of genes of interest which encode antigens which elicit an immune response in the recipient organism and which may be included in the plasmid in the transformed cell of the invention. An example of genes of interest which may be included in the plasmid to induce an immune response in the recipient organism are the cafI, caflA and caflM genes of the caf operon.

The gene of interest may be transcribed, or transcribed and translated, in the transformed cell prior to administration to a recipient organism or only following administration to a recipient organism depending on the identity of the promoter and the transformed cell. For example, where the gene of interest encodes an antigen which elicits an immune response functionally linked to a eukaryotic promoter and the transformed cell is a bacterial cell, the antigen will only be expressed to elicit an immune response following administration to the recipient organism.

In a further aspect of the invention, a composition for use as a medicament is provided comprising any of the cells described above together with a pharmaceutically acceptable excipient, diluent or buffer, such as are well-known to those of skill in the relevant art. According to one embodiment of this aspect, the pharmaceutical composition is a vaccine composition. The vaccine composition may comprise one or more adjuvants in additional to the excipient, diluent or buffer.

Other features and advantages of the invention will be apparent from the following description of the preferred embodiments thereof and from the claims.

### BRIEF DESCRIPTION OF THE DRAWINGS

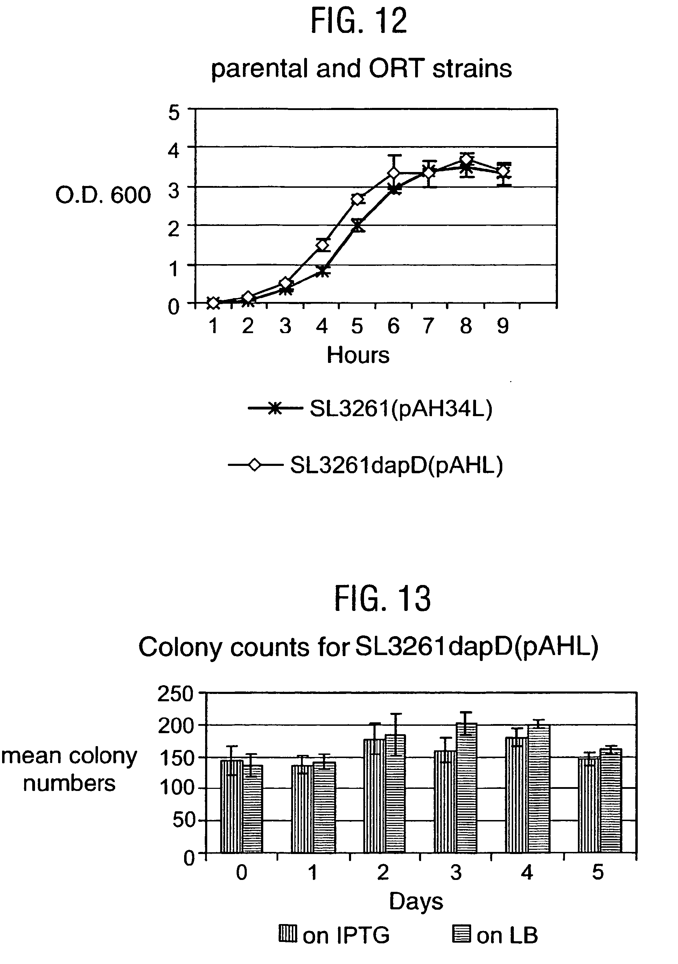

Figure 1 is a diagram illustrating the use of plasmid pKO3 (Figure 1A) for gene insertion (Figure 1B) from Link *et al,* 1997, J. Bacteriol 179: 6228-6237)
Figure 2A shows the chromosomal dapD locus, and B shows the PCR products generated from it for the cloning procedure.
Figure 3 shows maps of plasmids used in the construction of SL3261dapD (A: pTrc99; B: pGEM-T Easy; C: placPdapD6; D: pTrc99dapD; E: pGEM-TeasydapDL(-); F: pGEM-TeasydapDR(-); G: pDRdapD(+); H: pdapDB(+); I: pKO3; J: pKO3dapD)
Figure 4A shows the chromosomal fabA locus, and B shows the PCR products generated from it for the cloning procedure.
Figure 5 shows maps of the plasmids used in the construction of SL3261fabA (A: pCR2.1; B: pORT1; C: pCR2.1STfabA(-); D: pORfabA(+); E: placfabA; F: pTrc99fabA; G: pORIlacfabA; H: pK03fabA)
Figure 6 is a comparison of the growth curves of SL3261 in LB and of SL3261 dapD in LB and IPTG.
Figure 7: The plasmid pUC18I.
Figure 8: *In vitro* maintenance of pUC18I in SL3261dapD. Mean colony numbers of SL3261dapD(pUC18I) on LB-IPTG and on ampicillin are shown.
Figure 9: *In vitro* maintenance of pUC18I in SL3261dapD. Agarose gel of the mini-preps to show presence of pUC18I in SL3261dapD. M = kb ladder; C = pUC18I reference. The time points from 'day 0' to 'day 5' as described in Example 4 were loaded in chronological order. 1-2-3: SL3261 dapD(pUC 18I) clones.
Figure 10: *In vivo* maintenance of pUC 18I in SL3261 dapD in mice.
   First group of mice injected with SL3261 as a control: closed diamonds = total viable *Salmonella* detected in spleen cells plated on LB agar; open diamonds = total viable *Salmonella* detected in PP cells plated on LB agar.
   Second group of mice injected with SL3261 containing pUC18I: closed triangles = total viable *Salmonella* detected in spleen cells plated on LB agar; open triangles = *Salmonella* containing pUC18I detected in spleen cells plated on LB agar containing ampicillin; triangles with one bar = total viable *Salmonella* detected in PP cells plated on LB agar; triangles with multiple bars = *Salmonella containing* pUC18I detected in PP cells plated on LB agar containing ampicillin.
   Third group of mice injected with SL3261 dapD containing pUC 18I: closed circles = total viable *Salmonella* detected in spleen cells plated on LB agar and IPTG; open circles = *Salmonella* containing pUC18I detected in spleen cells plated on LB agar containing ampicillin; circles with one bar = total viable *Salmonella* detected in PP cells plated on LB agar with IPTG; triangles with multiple bars = *Salmonella containing* pUC18I detected in PP cells plated on LB agar containing ampicillin.
   Bacterial counts for the three groups of mice shown 8, 12 and 16 days after injection. Horizontal line across graphs just above 1 on vertical axis represents limit of detection
Figure 11: The plasmid pAHL.
Figure 12: Growth curves of SL3261(pAH34L) in LB-kanamycin and of SL3261dapD(pAHL) in LB.
Figure 13: *In vitro* maintenance of pAHL in SL3261dapD. Mean colony numbers of SL3261dapD(pAHL) on IPTG and on LB are shown.

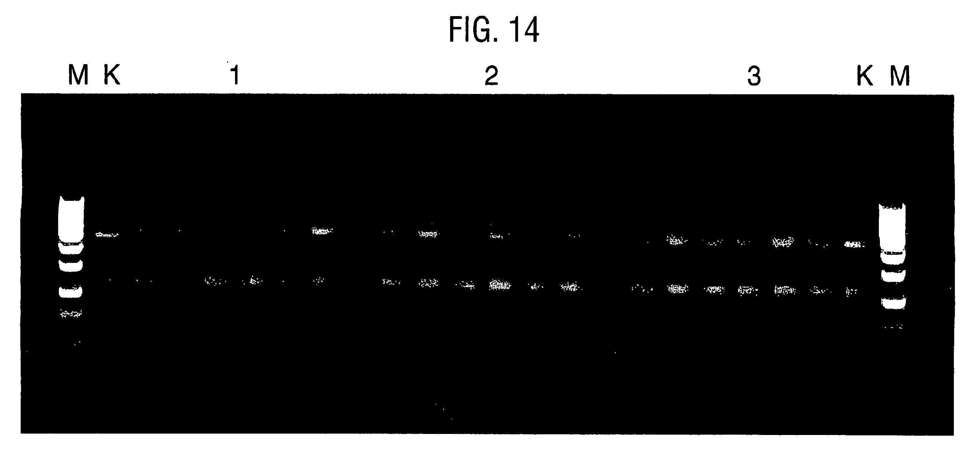

Figure 14: *In vitro* maintenance of pAHL in SL3261dapD. Agarose gel of the mini-preps to show presence of pAHL in SL3261dapD. M = kb ladder; K = pAHL reference. The time points from 'day 0' to 'day 5' as described in Example 7 were loaded in chronological order. 1-2-3: SL3261dapD(pAHL) clones.

### DETAILED DESCRIPTION OF THE INVENTION

The invention is illustrated by the following non-limiting examples wherein the following materials and methods are employed. The entire disclosure of each of the literature references cited hereinafter are incorporated by reference herein.

### Repressor/Operator Systems Useful According to the Invention.

The invention can be used with any *trans*-acting repressor/operator system. For example, the repressor titration system described herein may include any repressor that has a sufficient affinity for its DNA binding sequence such that it is capable of preventing expression of an essential chromosomal gene, but is also titratable by a plasmid-borne DNA binding sequence.

The essential chromosomal gene which is susceptible to repression by the repressor is rendered susceptible to repression in that it is placed under the control of an operator/promoter that binds the repressor, or the repressor binding sequence (i.e., operator) is inserted into or associated with the natural promoter of the essential gene in such a way that it can prevent transcription when bound by repressor, but does not disrupt the ability of the natural promoter to initiate transcription of the essential gene in the absence of repressor binding.

More than one different essential gene may be present in the chromosome, e.g., two or three genes, each gene being functionally linked to an operator sequence and thus susceptible to repression by the repressor. The presence of different repressor-susceptible essential genes on the chromosome, preferably at different positions in the chromosome, reduces the possibility of loss of plasmid stability via a mutation or deletion resulting in loss of repression of an essential chromosomal gene.

The repressor is encoded by a chromosomal gene. According to the invention, one or more, preferably one, two, or three, copies of the chromosomal repressor gene are present in the host cell. The chromosomal repressor gene may be a naturally occurring gene which has not been modified, or it may contain a genetic mutation that renders the repressor molecule of higher or lower affinity with respect to the strength of binding to its corresponding operator. Such mutations are known in the prior art, for example, the affinity of the lac repressor for its operator can be enhanced by single amino acid changes (see Betz, (1986) Gene, 42, 283-292 and Khoury et al., (1991) J. Mol. Biol., 219, 623-634). Alternatively, more or less copies of the operator binding site can be introduced into the plasmid or more or less copies of the repressor gene can be introduced onto the chromosome or in an alternative embodiment carried on a plasmid.

Alternatively, the sequences which initiate expression of the repressor gene such as promoters, enhancers etc. may be mutated or genetically engineered such that a higher or lower number of repressor molecules are made in the cell.

For example, the number of copies of the lacI repressor can be increased by the introduction of the lacI^{q} mutation (see Carlos (1978) Nature 274, 762-765). The number of repressor molecules present in the cell will be related to the copy number of the plasmid bearing the corresponding operator sequence. According to the repressor titration system of the invention, the concentration of repressor present in the host cell is such that, in the absence of the plasmid, the essential gene of interest is not expressed, but in the presence of the plasmid, repressor is titrated away from the essential gene. Where more than one copy of the repressor gene is present in the chromosome, e.g., two or three copies, the amount of repressor protein made in the cell will be increased relative to the presence of one gene; this increase will be taken into account when selecting a corresponding plasmid origin of replication, and in selecting the number of chromosomal operator/essential genes which are present in the cell.

The strength of the operators and the affinities of the repressors may be altered to tailor the system for use with plasmids of different copy number. For example, the extent of repression of the lac operon can be enhanced by the introduction of an optimally placed auxiliary ideal lac operator (i.e. a lac operator having enhanced repressor affinity), or the introduction of the ideal operator within the promoter (Muller et al., (1996) Mol. Biol., 257, 21-29 and Lewis et al., (1996) Science 271, 1247-1254).

Alternatively, the strength of the operator could be reduced by the introduction of a non-ideal operator, non-optimal positioning of the operator or elimination of an auxiliary operator (Muller et al., (1996) Mol. Biol., 257, 21-29 and Oehler et al., (1990) EMBO J. 219,973-979). For example, the affinity of the lac repressor for its operator can be enhanced by single amino acid changes (Betz, (1986) Gene, 42, 283-292).

### Repressor systems useful according to the invention

Repressor systems useful according to the invention include but are not limited to the following. The *E. coli lac* repressor is described in "The Lactose Operon", J. Beckwith, in *Escherichia coli* and *Salmonella typhimurium,* Eds., J.L. Ingraham et al., 1987 Amer. Soc. Micro., pp. 1444-1452, and Dickson et al., 1975, Science 187:27-35.

The lac operon is regulated as follows. Under non-inducing conditions (such as growth on glucose) LacI binds to the operator of the lac operon and prevents transcription of β-galactosidase (LacZ), lactose permease (LacY) and a transacetylase (LacA). Under inducing conditions (such as growth on lactose or addition of IPTG, a non-metabolisable analogue) the repressor no longer binds to the operator and transcription occurs. The expression of the operon is easily detected by assay for β-galactosidase. Other repressor systems useful according to the present invention include the lac repressor system described above and the tet repressor system for use in regulating gene activity in eukaryotic cells (Gossen et al., (1994) Current Opinions in Biotechnology, 5, 516-520). The tet repressor system has been used in yeast, *Dictyostelium,* plant cells and tobacco plants. A further repressor system useful according to the present invention is the ArgRNV repressor system (Burke et al., (1994) Mol. Microbiol. 13, 609-618). The ArgR repressor normally only binds to its operator in the presence of arginine. However, the mutant ArgRNV repressor binds to the operator in the absence of arginine and remains bound in the presence of arginine and has a transdominant effect. An idealised ArgR binding site (operator) having two symmetrical Arg boxes, can be engineered into the plasmid of interest to enable the titration of ArgRNV away from an essential gene the expression of which is controlled by the ArgR binding site.

One of skill in the art will appreciate that certain modifications may be made to the repressor-titration system described herein which serve to adapt the system to a given protocol. For example, where the growth medium contains components which induce rather than allow for repression of the operator, and inducing conditions are not desired during growth, operator or repressor mutants may be used to overcome induction and allow for repression. One example of a mutant repressor is a LacI mutant of the lac repressor. A LacI mutant no longer has the capacity to bind inducer. Examples of LacI mutants include, e.g., LacI mutants (Beyreuthe, 1978, Cold Spring Harbor Laboratory, CSH, NY) and other mutants such as Asp276 - > Asn274 (Chang et al., 1994, Biochem. 10 22:3607-3616). By replacing the wild type repressor with a mutant repressor which is insensitive to inducer, the repressor is able to bind to the operator during growth, and the plasmid is maintained in the host cell even under conditions which normally induce the repressor.

The *E. coli* trp repressor also is useful according to the invention (see "The tryptophan Operon", Yanofsky and Crawford, in *Escherichia coli* and *Salmonella typhimurium,* Eds., J.L. Ingraham et al., 1987, Amer. Sec. Micro., pp. 1453-1472). The trp repressor is present at about 50 copies/cell, and requires the presence of tryptophan in the fermentation medium as an inducer of repressor binding. 20

The *E. coli* gal R repressor also is useful according to the invention (see "The Galactose Operon", S. Adhya, in *Escherichia coli* and *Salmonella typhimurium,* Eds., J.L. Ingraham et al., 1987, Amer. Soc. Micro., pp. 1503-1512).

The *E. coli* ara C repressor is also useful according to the invention (see "The L-Arabinose Operon", R. Schlief, In *Escherichia coli* and *Salmonella typhimurium,* Eds., J.L. Ingraham et al., 1987, Amer. Soc. Micro., pp. 1473-1481; Dunn et al., 1984, Proc. Nat. Aca. Sci. 81 ;5017-5020). The ara C repressor has increased binding affinity in the presence of arabinose. Finally, the A repressor is useful according to the invention (Introduction to Lambda Phages, in Current Protocols in Molecular Biology, Eds. Ausubel, et al., 1994, Section III, Unit 1.9; Hochschild et al., 1986, Cell 47(5);807-816).

### Plasmids Useful According to the Invention

The invention can be utilised advantageously with a plasmid origin of replication that permits replication of at least 5, preferably at least 10-100, and most preferably at least 100-500 copies of the plasmid per host cell. Those origins of replication that permit replication of moderate (i.e., 20-50) to high plasmid (i.e., 100-500) copy numbers are especially useful in that moderate to high plasmid copy numbers can easily titrate repressor molecules. Of course, if desired, a plasmid having a copy number as high as 1000-2000 copies per cell also may be used.

Plasmids with low copy numbers (i.e., 5 copies or fewer) are most advantageously used according to the invention after mutation to bring about increased copy number (J. Scott, 1984, Microbial Reviews 48:1-23). Of the frequently used origins of replication, pBR322 (20 copies/cell) is useful according to the invention, and pUC (at 200 copies/cell) are preferred. Although not preferred, other plasmids which are useful according to the invention are those which require the presence of plasmid encoded proteins for replication, for example, the pT181, Fll, and Fl origins of replication.

Examples of origins of replication which are useful according to the invention in *E. coli* and *S. typhimurium* include but are not limited to pMB1 (25 or more copies per cell, 20 Bolivar et al., 1977, Gene 2:95-113), ColEI (15 or more copies per cell, Kahn et al., 1979, Methods Enzymol. 68:268-280), p15A (about 15 copies per cell, Chang et al., 1978, J. Bacteriol. 134:1141-1156); pSC101 (about 6 copies per cell, Stoker etal., 1982, Gene 18:335-341); R6K (less than 15 copies per cell, Kahn et al, 1979, supra); R1 (temperature dependent origin of replication, Uhlin et al, 1983, Gene 22:255-265); lambda dv (Jackson et al., 1972, Proc. Nat. Aca. Sci. 69:2904-2909). An example of an origin of replication that is useful in Staphylococcus is pT181 (about 20 copies per cell, J. Scott, 1984, Microbial Reviews 48:1-23). Of the above-described origins of replication, pMBI, p15A and ColEI are preferred because these origins do not require plasmid-encoded proteins for replication.

### Host Cells Useful According to the Invention.

The invention is applicable to all cell types including animal cells such as mammalian and insect cells, plant cells, fungi such as yeast, and most strains of bacteria, for example, gram positive and negative bacterial strains, provided a plasmid exists that is capable of being maintained in the host cell at a medium to high copy number.

Gram negative bacteria useful according to the invention include but are not limited *to E. coli* and *Salmonella,* e.g., *S. typhimurium.*

Gram positive species useful according to the invention include but are not limited to *Bacillus, Streptomyces, Lactobacillus* and *Lactococcus,* for which high copy number plasmids already exist. Examples of plasmids useful according to the invention in Lactococcus are pNZ2123 and p1L253 (Simon et al., Biochimie 70:559,1988). The lactococcal lactose operon has been used to control the expression of heterologous proteins (Wells et al., 1993, Mol. Microbiol. 8(6):1155-1162). This operon utilises the lacR repressor (van Rooigen et al., J. Biol. Chem. 265:18499-18503, 1990) to control the expression of T7 polymerase, which then controls the expression of the heterologous protein. Examples of plasmids useful according to the invention in *Bacillus* are pC 194, pUB110 and pT181. In *Bacillus,* e.g., *B. subtilis,* the A repressor has been used to control the expression of heterologous proteins. The A repressor has been placed under the control of the sak42D promoter, which can be efficiently transcribed in *B. subtilis* (Breitling et al., 1990, Gene 93(1):35-40).

Yeasts are useful according to the invention, as high copy number plasmids are maintained in yeasts. Examples of such plasmids include the YRp plasmids (based on autonomously replicating sequences (ARS)) which have copy numbers up to about 100 copies per cell, and the YEp plasmids (based on the 2 micron circle), with a copy number of 50-100 per cell. (See Sikorski, "Extrachromosomal cloning vectors of *Saccharomyces cerevisiae",* in Plasmids, A Practical Approach, Ed. K.G. Hardy, IRL Press, 1993; and Yeast Cloning Vectors and Genes, Section II, Unit 13.4, Current Protocols in Molecular Biology, Eds., Ausubel et al., 1994.) Yeasts are able to express the *E. coli* lacZ gene, it is therefore contemplated according to the invention to use the lac repressor titration system to control the expression of essential yeast genes such as ura3 or Ieu2, genes which have been used for the maintenance of plasmids in yeasts (Gunge, 1983, Ann. Rev. Micro. 37:253-276).

Particularly useful are attenuated host cells that have been attenuated so as to be acceptably free of side-effects free for therapeutic use in recipient organisms. Depending on the specificity of the cell, it may be suitable for veterinary or human therapeutic use.

Among the attenuated bacteria available are the following:
*Salmonella typhimurium* including strains SL3261 (aroA mutant, Titball et al, 1997, Infection and Immunity 65: 1926, Titball et al, 1995, Infection and Immunity 63: 563), VNP20009 (purl and msbB mutant, Toso et al, 2002, J Clin Oncol 20: 142);
*Salmonella typhi* including strains CVD 908-htrA, χ4073, χ4632, Ty800, CVD 909 and CVD 915 (Galen et al, 1999, Infect. Immun. 67: 6424-6433; Morona et al, 1991, Gene 107: 139-144; Tacket et al, 1997, Infect. Immun. 65: 3381-3385; Garmory et al, 2002, FEMS Microbiology Reviews 26: 339-353);
*Salmonella gallinarum* including strain 9R (for avian vaccination, Feberwee et al, 2001, Avian Dis 45: 1024);
*Escherichia coli* including aroA, cnfl and OMP mutants (Rippere-Lampe et al, 2001, Infect Immunol 69: 3954);
*Shigella flexneri* including strain *S.flexneri* 2a guaBA (Altboum et al, 2001, Infect Immunol 69:3150);
*Vibrio cholerae* including strain Peru2 (Ryan et al, 2000, Infect. Immun. 68: 221-226);
*Listeria monocytogenes* including PrfA and SvpA mutants (Sheehan et al, 1996, Mol Microbiol 20: 785; Borezee et al, 2001, Microbiology 747:2913);
*Brucella abortus* including strain RB51 (Vemulapalli et al, 2000, Infect Immunol 68:3290);
*Mycobacterium bovis* including the strain Calmette-Guerin (BCG); and
*Mycobacterium tuberculosis.* Auxotrophs of M tuberculosis are known that might be used directly for the ORT^{®} approach, such as the trpD mutant (tryptophan auxotroph, Smith et al, 2001, Infect Immun 69: 1142).

### Essential Genes Useful According to the Invention.

The invention may be used in conjunction with a number of different essential chromosomal host genes for the stable maintenance of the plasmid. These essential genes include but are not limited to the following categories, e.g., genes encoding products related to the biosynthesis of cell metabolites, genes whose products are involved in carbon metabolism, genes coding for antibiotic resistance, and genes encoding the biosynthesis or regulation of macromolecules, e.g., genes essential for DNA and/or RNA synthesis and replication functions.

### 1. Essential genes encoding products related to synthesis of components of cell structure.

Certain genes encoding enzymes involved with the supply of cell components, in particular the supply of cell wall precursors, are also essential for host cell growth and are useful according to the invention. For example, the bacterial cell wall contains meso-diamiopimelic acid (DAP), and an inability to synthesise this component results in cell lysis.

It has been demonstrated that mutants in which the asd gene (aspartate p-semialdehyde dehydrogenase) or dapD gene (tetrahydrodipicolinate N-succinyl transferase) are deleted can be used for the maintenance of plasmids that carry a complete copy of that gene on the plasmid. (Nakayama et al., Bio/technology 6:693-697, 1988; DeGryse, U.S. Pat. No. 5,198,343).

A number of other genes in the DAP biosynthetic pathway could also be used, namely dapA, dapB, dapC and dapE genes. dapA and dapB have been cloned and sequenced, and dapB is available as a single cistron (Richaud et al., J. Bacteriol. 166:297-300, 1986; Bouvier et al., J. Biol. Chem. 259:14829-14834, 1984).

The genes involved in the biosynthesis of other cell wall components, such as D-alanine biosynthesis, are also useful according to the invention (Walsh, 1989, J. Biol. Chem. 264(5):2393-2396). A DNA sequence encoding a component for D-alanine has been used for the stabilisation of plasmids without using antibiotics (see EP 85/309020).

Genes involved in fatty acid biosynthesis are also useful according to the invention. One example is fabA, which encodes 3-hydroxydecanoyl-ACP dehydrase, responsible for the introduction of a double bond into the growing fatty acid chain at the point where unsaturated and saturated fatty acid biosynthesis diverges (Cronan Jr.J.E. and Rock.C.O. (1987)). Biosynthesis of membrane lipids. In *'Escherichia coli* and *Salmonella typhimurium:* cellular and molecular biology'. Neidhardt .F.C. (ed.) American Society of Microbiology, Washington D. C.). The enzyme is present at relatively high concentration, and fabA mutants lyse unless supplemented with an unsaturated fatty acid.

The invention contemplates the use of repressor titration in conjunction with such genes. The wild-type gene of interest is deleted from the host strain and replaced with a copy in which expression is directed by a promoter/operator that binds the chosen repressor protein, using a host strain that synthesises the repressor protein. Transformation of the strain with a plasmid containing the repressor binding sequence therefore results in titration of the repressor away from the biosynthetic gene, enabling expression of the essential gene.

### 2. Genes essential for cell growth

The repressor-titration method of the invention can be used with genes involved with the utilisation of carbon sources. Specifically, the method can be used with the lactose operon and the utilisation of lactose as the sole carbon source, as described herein. Other modifications will be apparent to one of skill in the art. Mutants of the lac repressor exist that are no longer able to bind the inducer (allolactose) and remain bound to the lac operator in normal inducing conditions. These are typified by the lac Is mutants; however, other mutations exist that have no capacity to bind inducer but are normal in all other functions (Chang et al., Biochem. 33:3607-3616 (1994)).

Strains carrying these mutations would not be able to express the genes of the lac operon and hence not be able to grow with lactose as the sole carbon source. Transformation of such strains with high copy number plasmids containing wild type lac operator sequences will titrate the repressor away from the lac operon and allow growth on lactose.

Glutamine synthetase is an essential gene for eukaryotic cells such as the NSO myeloma cell line (Bebbington et al., (1992) Bio/Technology 10, 169-175) and is preferably used when the host cell is a eukaryote cell.

### 3. Genes encoding the synthesis of nucleic acids

The invention can also be used in conjunction with essential genes encoding DNA and/or RNA synthesis or replication proteins of the host cell. Examples of such genes with respect to these essential functions in bacteria such as *E. coli* and *Salmonella* are provided in McMacken et al. (in *Escherichia coli* and *Salmonella typhimurium,* Cellular and Molecular Biology, Ed. Neidhardt et al., Amer. Soc. Micro., Wash. D.C., 1987, pp.564-612), and include but are not limited to the following genes: dnaA, dnaB, dnaC, ssb, dnaG, polC (dnaE), dnaQ (mutD) dnaN, dnaZX, gyrA, gyrB, polA, lig, dnaT, rpoA, rpoB, rpoC, and rpoD.

### 4. Genes conferring a selectable advantage for survival in a recipient organism

For host cells administered to a recipient organism, depending on the route of administration a number of factors in the local environment may exert selective pressure. Where an ability to resist such pressure is linked to an identified gene, then this may be used as a selectable marker. For instance, for cells, especially bacterial cells to be administered orally, resistance to the low pH of the stomach contents constitutes a significant selective advantage. Among the genes shown to be associated with this are the uvrA gene of *Streptococcus mutans* (Hanna et al, 2001 J Bacteriol 183: 5964), the gadA, B and C genes of *E. coli* (Castanie-Comet and Foster, 2001, Microbiology 147: 709), the rpoS, fur, atp and atbR genes of *Salmonella typhimurium* (Baik et al, 1996 Microbiology 142: 3195), the lepA, Frasel, czcA, uvrA, atpF' and aldo-keto reductase genes of *Helicobacter pylori* (Bijlsma et al, 2000, J Infect Dis 182: 1566) and the genes encoding the F₀F₁ ATPase of *Listeria monocytogenes* (Cotter et al 2000 Int J Food Microbiol 60: 137).

Genes conferring intracellular survival (for instance SvpA in *Listeria monocytogenes* Borezee et al, 2001, Microbiology 147:2913) or resistance to particular intracellular locations such as lysosomes might also be useful in appropriate host cells.

### 5. Genes encoding antibiotic resistance

Although not preferred, the invention can also be used in conjunction with antibiotic resistance. The resistance gene is constructed such that its expression is under the control of the promoter/operator that binds the desired repressor protein. This construct is then inserted into the chromosome of the host strain. Transformation of the strain with plasmid containing the repressor binding sequence will titrate the repressor from the antibiotic resistance gene and allow expression and hence growth in the presence of that antibiotic. An antibiotic resistance gene is such a useful selectable marker that a practitioner of the invention might choose antibiotic resistance as the host essential gene even though attention would have to be paid to the systemic effects on the recipient. In this case the advantage of using the operator repressor titration approach lies in the capacity of the plasmid. The fact that the antibiotic resistance gene itself is encoded on the host cell chromosome increases the size of insert that may be cloned into the ORT® expression plasmid.

### Plasmid-borne genes useful according to the invention

The gene of interest borne on the plasmid in the transformed attenuated cells of the invention may be any gene. The gene may be one which it is desired to deliver to the recipient organism due to its therapeutic effect. For example, it may be desirable to deliver the gene to the recipient organism for gene therapy purposes.

Alternatively, it may be desirable to deliver the gene to the recipient organism because the transcription product or the transcription and translation product of the gene has a therapeutic effect. For example, the transcription product of the gene of interest may be an antisense oligonucleotide or the gene of interest may encode an antigen which has a therapeutic effect. In particular, the gene of interest may encode one or more antigens which elicit an immune response in the recipient organism. An example of genes of interest which may be included in the plasmid to induce an immune response in the recipient organism are the cafI, caflA and caflM genes of the caf operon.

Where it is desired that the gene of interest be transcribed or transcribed and translated, it is preferably functionally associated with a promoter. Preferably, the promoter is a eukaryotic promoter or a bacterial promoter. The choice of the promoter functionally associated with the gene of interest and the choice of the host cell determines whether the gene of interest is transcribed, or transcribed and translated, in the host cell prior to administration to the recipient organism or only after administration to the recipient organism.

In some cases, it may be desirable to deliver host cells already expressing a gene of interest, in which case, the promoter functionally associated with the gene of interest will be chosen such that it is operational in the host cell and promotes transcription and translation of the gene of interest in the host cell. For example, where the host cell is a bacterial cell, the promoter functionally associated with the gene of interest in this situation may be a bacterial promoter.

In other cases, it may be desirable to deliver host cells comprising the plasmid comprising the gene of interest to the recipient organism but to delay expression of the gene of interest until after delivery of the cells to the recipient organism. For example, where the host cell is a bacterial cell, the promoter functionally associated with the gene of interest in this situation may be a eukaryotic promoter which will not promote transcription and translation of the gene of interest in the host cell but will do so following administration to a recipient organism.

### Methods of vaccination useful according to the invention

It is envisaged that vaccination with recombinant live attenuated cells according to the invention might be by any of a number of routes of administration. For *Mycobacterium,* for instance the usual route is by intradermal or subcutaneous injection. For enteric bacteria such as *Salmonella, Shigella* or *E coli,* oral administration (particularly of acid-resistant transformants) or rectal administration is preferable. In some circumstances, mucosal, intravenous or intraperitoneal administration might be preferred.

### Administration for other in vivo applications

It is envisaged that a number of methods of administration for non-vaccination applications might be used.

In addition to the enteral and parenteral routes of administration used for vaccination, non-vaccine application might require other procedures. Amongst these are the use of implantable permeable containers in which cells according to the invention are contained.

### EXAMPLES

### Example 1: Conversion of Salmonella typhimurium SL3261 to an dapD ORT^{®} strain

The *Salmonella typhimurium* aroA mutant SL3261 (Oyston et al, 1995, Infect. Immun. 63:563-568; Titball et al, 1997, Infect. Immun. 63: 563-568) is converted to a strain that facilitates plasmid maintenance by operator-repressor titration (ORT^{®}). This involves replacing an essential chromosomal gene with that same gene under the control of the lac operator/promoter (lacO/P; Williams et al, 1998, Nucleic Acids Res. 26: 2120-2124). The gene replacement is carried out using a strategy that does not leave an antibiotic resistance gene on the SL3261 chromosome.

### Essential dapD gene controlled by lacO/P

The gene dapD encodes tetrahydrodipicolinate N-succinyl transferase which catalyses the conversion of tetrahydrodipicolinate and succinyl-CoA to Nsuccinyl-α-amino-ε-ketopimilate and CoA in the lysine/diaminopimelate pathway (Richaud et al, 1984, J. Biol.

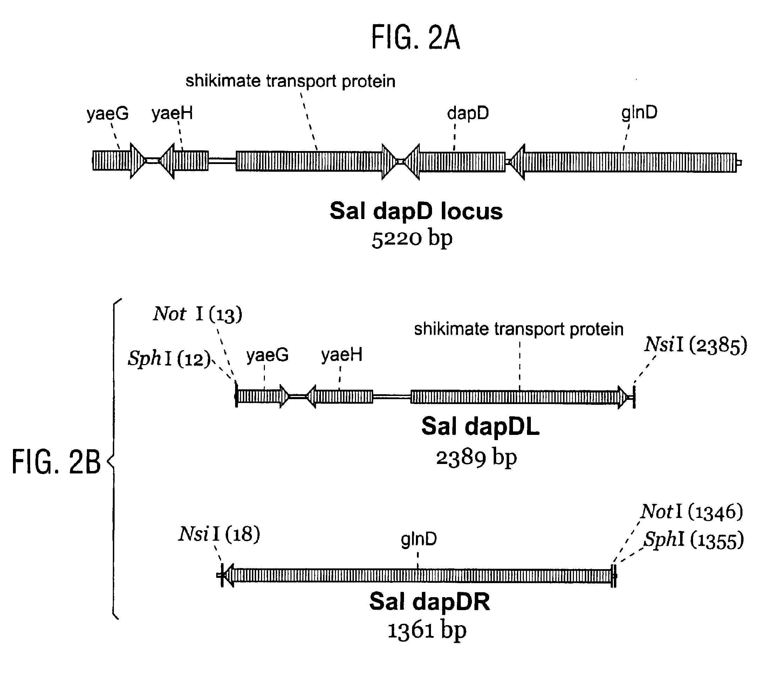

Chem, 259:14824-14828; Gilvarg, 1961, J. Biol. Chem, 236: 1429-1431). The chromosomal dapD locus is shown in Figure 2A. DAP cross-links peptidoglycan in the bacterial cell wall and is a precursor for lysine biosynthesis (Work, 1950, Nature 765: 74-75). Mutants with disrupted dapD are therefore DAP and lysine auxotrophs. As lysine is present in the rich media often used to grow *E. coli* and *S. typhimurium* (such as LB broth) and DAP is not, dapD mutants will undergo cell lysis unless DAP is added to the medium (Davis, 1952, Nature 169: 534-536). dapD has already been successfully used as the ORT^{®} gene in *E. coli* strains DH1*lacdapD* and DH1*lacP2dapD* (Cranenburgh et al, 2001, Nucleic Acids Research 29: E26). Therefore the *E. coli lac-dapD* constructs are used to complement *dapD* in *S. typhimurium.*

### Creating the lac-gene fusion constructs

The construct with the *E.coli dap*D under the control of the mutated, non-leaky *lac* promoter (*lac*P2) are present in the plasmid placPdapD6. The 3' end of the lactose repressor gene *lacI* is already present in these constructs, so the full-length gene must be cloned in. This is necessary since *lacI* is either absent or not functional in *S. typhimurium* SL3261 (Oyston et al, 1995, Infect. Immun. 63:563-568 and confirmed by inventors). The source of this gene is the plasmid pTrc99 (Amersham-Pharmacia), which has the over-expressing mutation lacI^{q}.

### Creating the gene replacement cassette

The regions flanking *dapD* from *S. typhimurium* were amplified by PCR, and these products (shown in Figure 2B) cloned into separate pGEM-T Easy plasmids (Promega). These left and right flanks were then cut out and ligated into a suitably prepared pTrc99dapD (which has *lacO*/*P-dapD* next to *lacI^{q}*), either side of the expression cassette (i.e. flanking *lacI^{q}* and *lacO*/*P-dapD*) and in correct orientation relatively to the chromosomal dapD. This procedure created the plasmid pdapDB(+).

### Insertion of the ORT^{®} cassette into the SL3261 chromosome

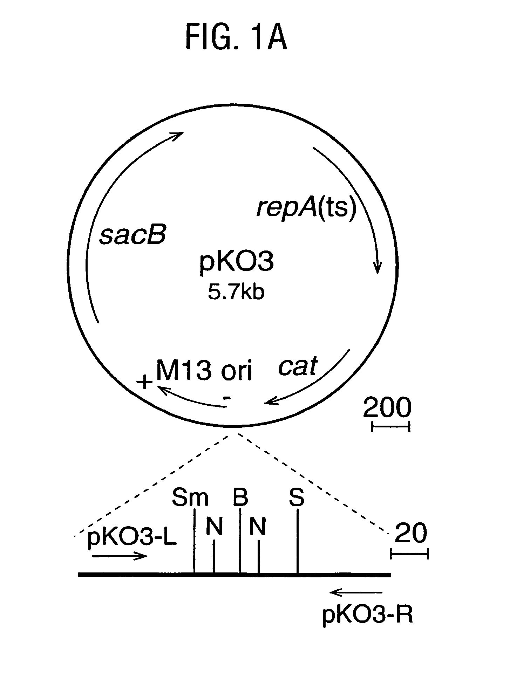

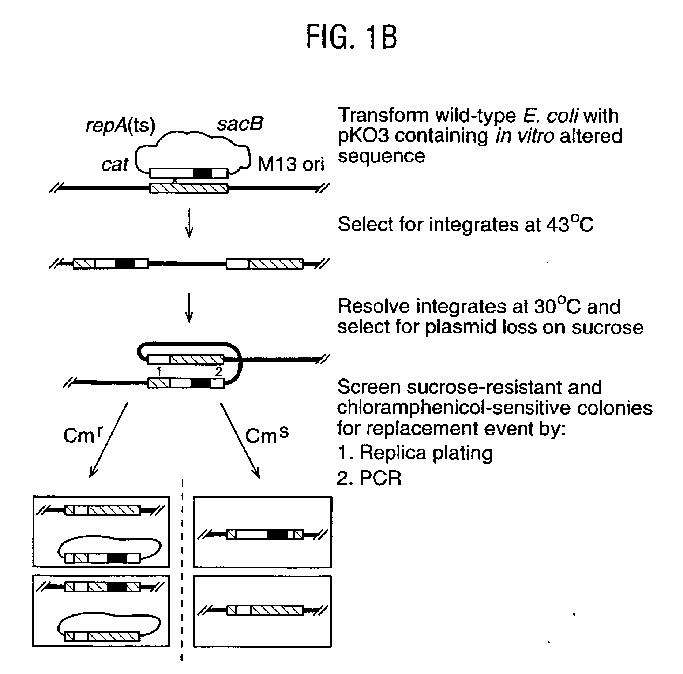

The integration plasmid is based on pKO3 (Link, 1997, J. Bacteriol 179: 6228-6237), which utilises the levansucrase gene *sacB* from *Bacillus subtilis* as a counter-selection. *sacB* converts sucrose into a toxic compound that will kill cells containing pKO3. The gene replacement cassette is subcloned from pdapDB+ into the polylinker of pKO3, then the chloramphenicol resistance gene cat is the selection for the initial chromosomal integration via a single recombination event. The next step is the selection for the resolution of the cointegrant in the presence of sucrose to yield the removal of the pKO3 sequences from the chromosome, leaving only the *lac*-gene fusion. The principles of this procedure are shown in Figure 1.

### Summary

1. *Lac*-gene fusions are cloned into pTrc99 adjacent to *lacI^{q}.*
2. Amplify the regions flanking *dapD* from *S. typhimurium* chromosomal DNA by PCR.
3. Assemble the deleted gene loci.
4. Clone the deleted gene loci PCR products into pGEM-T Easy.
5. Cut out the *dapD* gene flanks and ligate either side of *lacl^{q}* and *lac O*/*P-dapD.*
6. Cut out the deletion cassette and ligate into the multi-cloning site of pKO3.
7. Use this pKO3-based plasmid to insert the *lacI^{q} + lac-dapD* cassette into the chromosomal dapD locus, deleting the wild-type gene in the process.
8. Verify ORT^{®} strain genotype by PCR and Southern analysis, and test plasmid selection and maintenance by ORT^{®}.

### Detailed cloning strategy for an dapD ORT® strain of Salmonella typhimurium SL3261

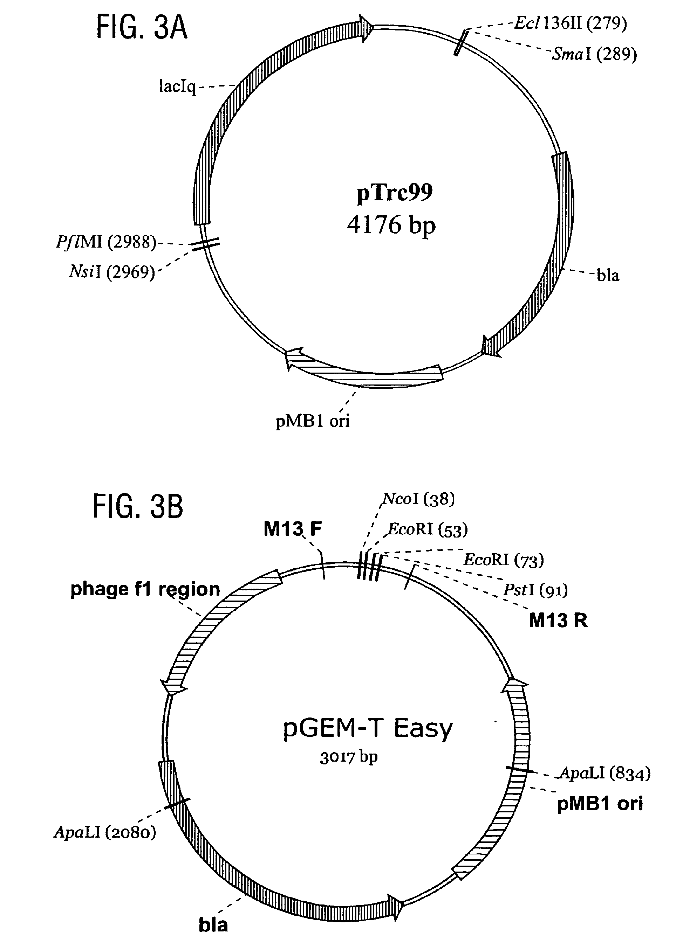

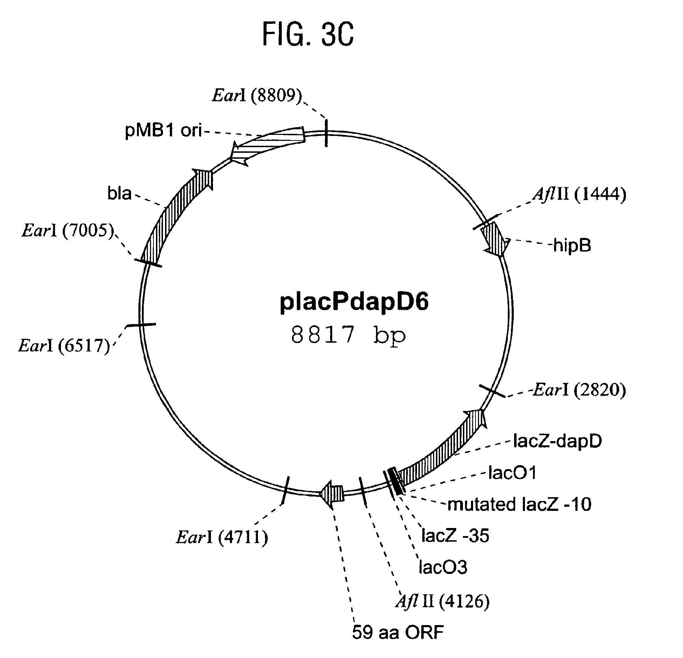

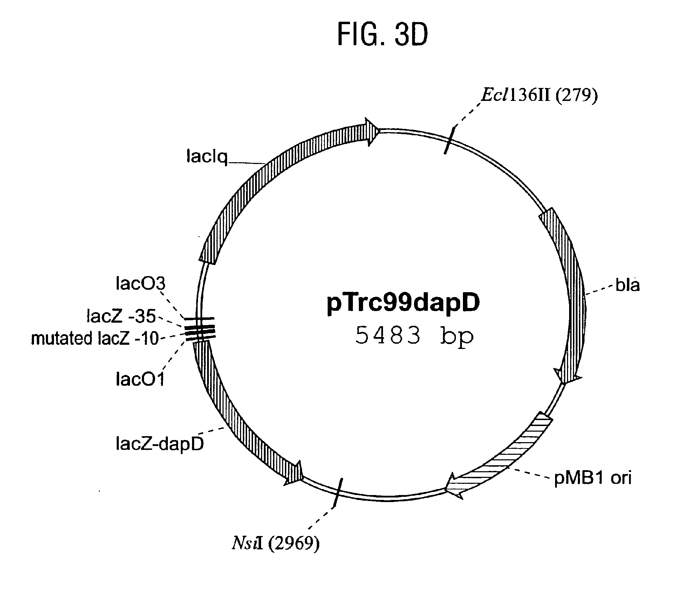

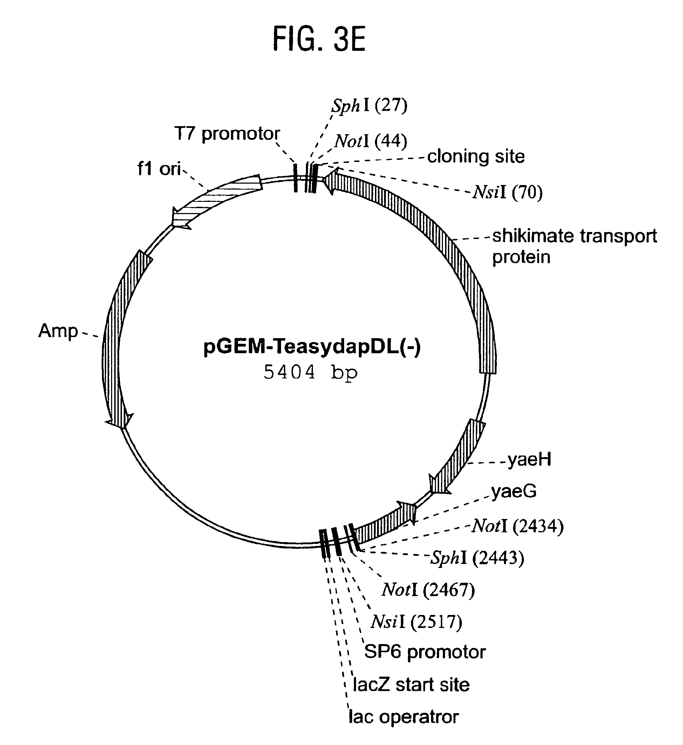

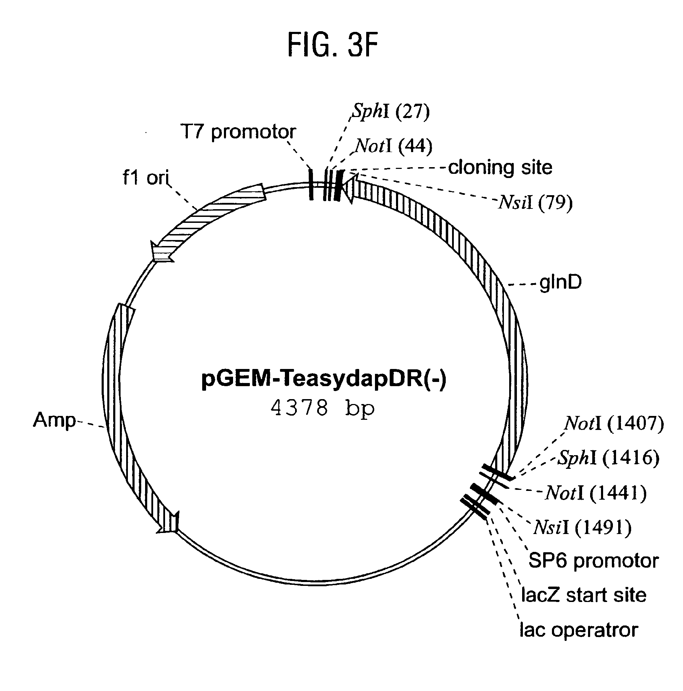

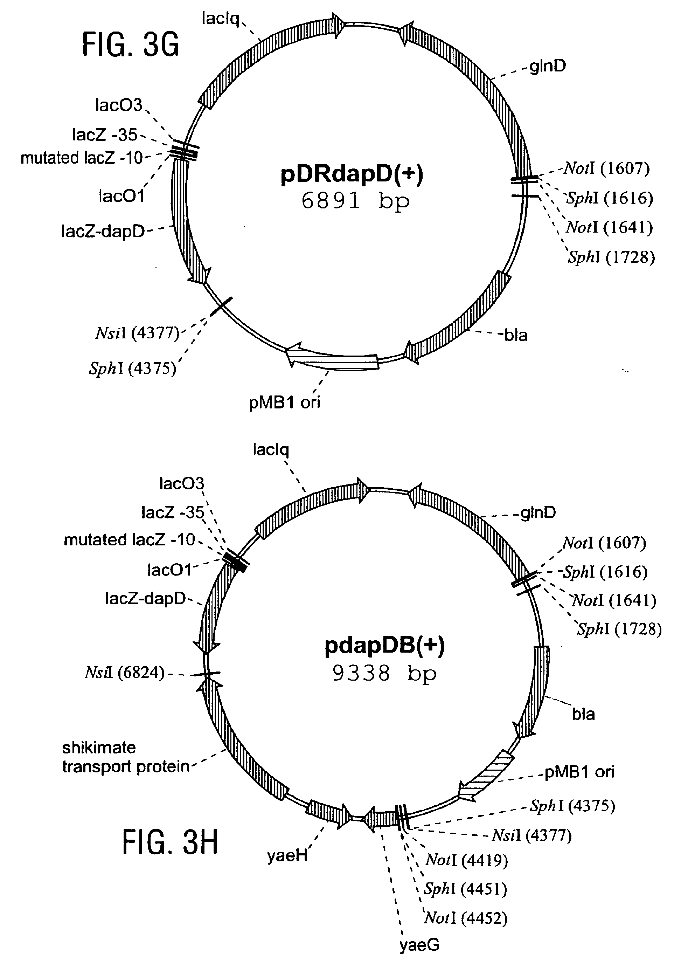

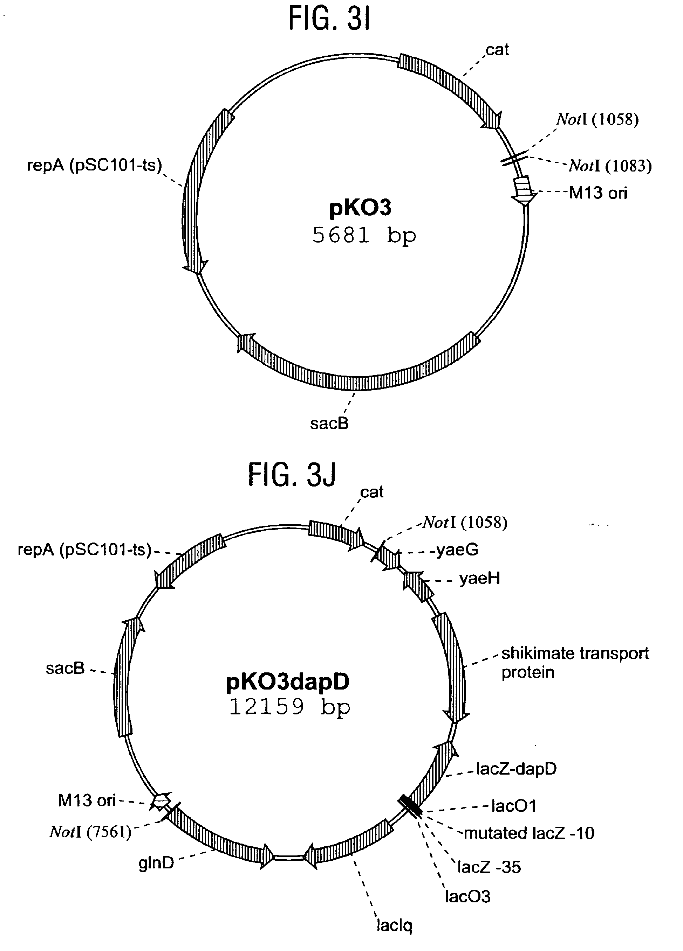

1. Cut pTrc99 (Figure 3A) with *Pfl*MI and blunt ends (with T4 DMA polymerase).
2. Remove the *E. coli lacZ-dapD* expression cassette from placPdapD6 (Figure 3C) as an *Afl*II*-Ear*I fragment and blunt ends.
3. Ligate to create pTrc99dapD (Figure 3D). Orientations of dapD and *lacl^{q}* must be opposite.
4. Amplify the 5' and 3' flanks of *dapD* from *Salmonella typhimurium* SL3261 chromosomal DNA by PCR using primers 5STdapD with LST2dapD (PCR product = 2406 bp) and RSTdapD with 3STdapD (PCR product = 1361 bp). The flanking primers (5STdapD and 3STdapD) contain *SphI* and *NotI* sites.
5. Clone both flanks into pGEM^{®}-Teasy (Promega) shown in Figure 3B to create pGEM-TeasydapDL(-) (Figure 3E) and pGEM-TeasydapDR(-) (Figure 3F).
6. Cut the right flank out of pGEM-TeasydapDR(-) (Figure 3F) as an *Nsi*I fragment, blunt it and ligate it into pTrc99dapD (Figure 3D) cut with *Ecl*1136II to create pDRdapD(+) (Figure 3G). Then cut the left flank out of pGEM-TeasydapDL(-) (Figure 3F) as an *Nsi*I fragment and ligate it into pDRdapD(+) (Figure 3G) cut with *Nsi*I to create pdapDB(+) (Figure 3H).
7. Cut out the insert from pdapDB(+) (Figure 3H)as a *Not*I fragment and ligate into *Not*I-cut pKO3 (Figure 3I) to create pKO3dapD (Figure 3J) (insert orientation not important).
8. Use pKO3dapD (Figure 3J) to replace wild-type *dapD* with *lacZ-dapD* in *S. typhimurium* SL3261. The resulting strain is SL3261 dapD.

### PCR primers used.

*Sph*I and *Not*I sites in bold, *Nsi*I sites
in underlined.
5STdapD (39 nt)
CGAATGGCATGCGGCCGCAGCTATTTTTATCAGGATTTG
LST2dapD (35 nt)
GCGAATGCATAGCGGGGGCGACAGCCCCCCTTTGC
3STdapD (39 nt)
TGTGGGGCATGCGGCCGCAGTGGTCGCACATCGTCGGAC
RSTdapD (46nt)
GCTTTTCACTCGATGCATCTACCCTTTATCGTTTGGATTGAGGGCC

### Example 2: Conversion of Salmonella typhimurium SL3261 to a fabA ORT® strain

### Essential fabA gene controlled by lacO/P

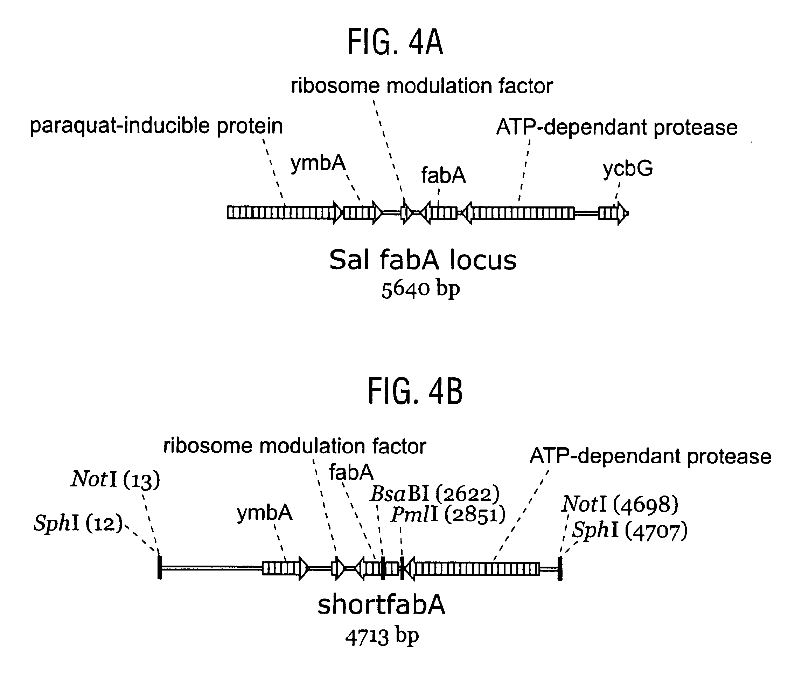

A alternative gene suitable for use in an ORT^{®} stain is fabA, encoding 3-hydroxydecanoyl-ACP dehydrase, which catalyses the introduction of a double bond in the growing fatty acid chain at the point where unsaturated and saturated fatty acid biosynthesis diverges (Cronan and Rock, 1987, Biosynthesis of membrane lipids. In *'Escherichia coli* and *Salmonella typhimurium:* cellular and molecular biology'. Neidhardt,F.C. (ed.). American Society of Microbiology, Washington DC). The chromosomal fabA locus is shown in Figure 4A. The enzyme is present at a relatively high concentration, and fabA mutants lyse unless supplemented with an unsaturated fatty acid, even in LB medium. This makes fabA a suitable choice for an ORT® strain.

A similar procedure to that explained in Example 1 is used to generate a fabA ORT® strain, but rather than amplify the two gene flanking regions as separate PCR products, a single region containing the entire fabA locus was amplified by PCR and cloned. fabA was then cut out and replaced with the *lacl^{q}* and *lacO*/*P-fabA* cassette. The PCR product generated from the fabA locus for the cloning procedure is shown in Figure 4B.

### Detailed cloning strategy for generating ORT® strain based on fabA

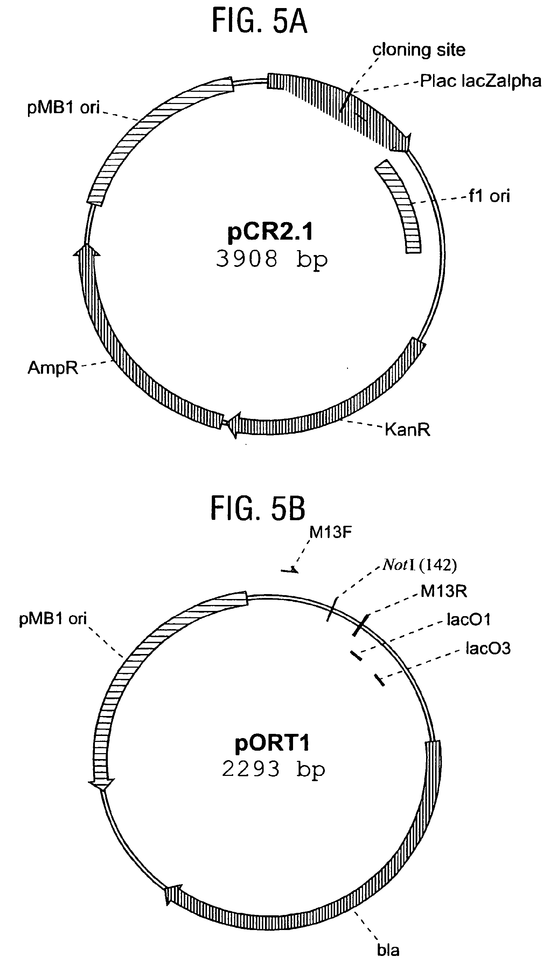

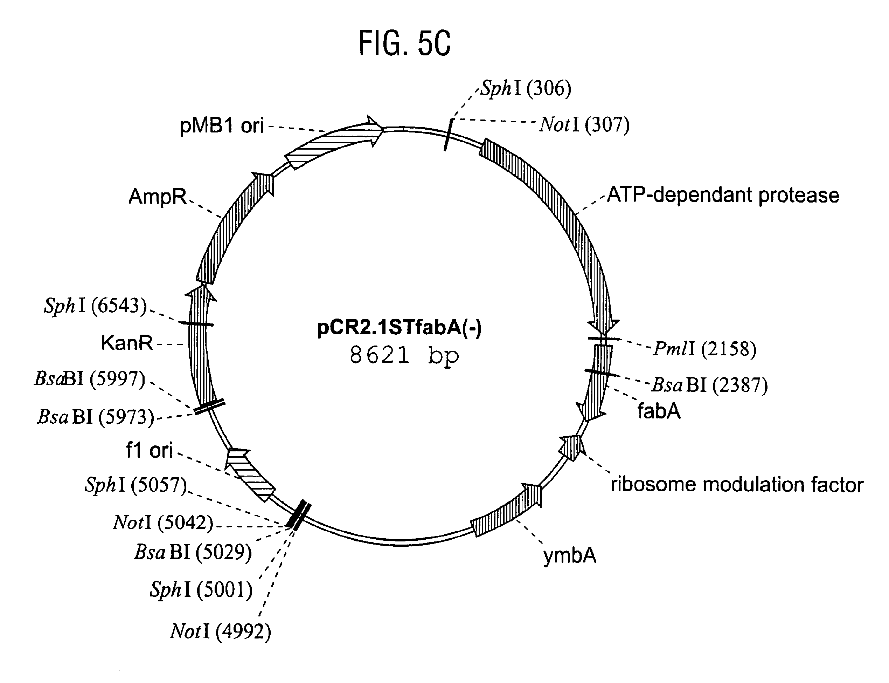

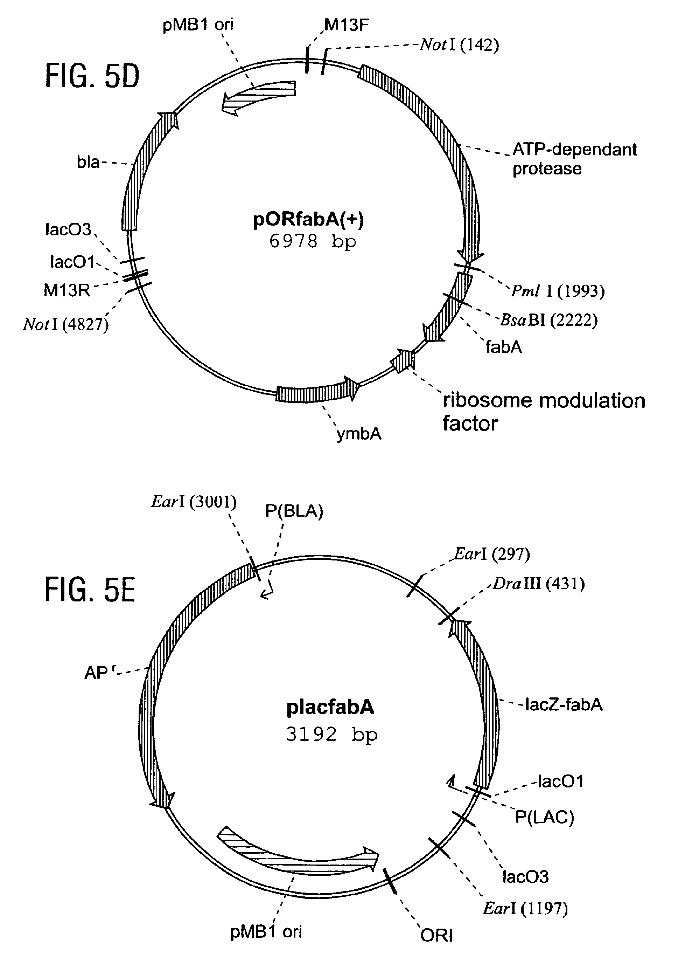

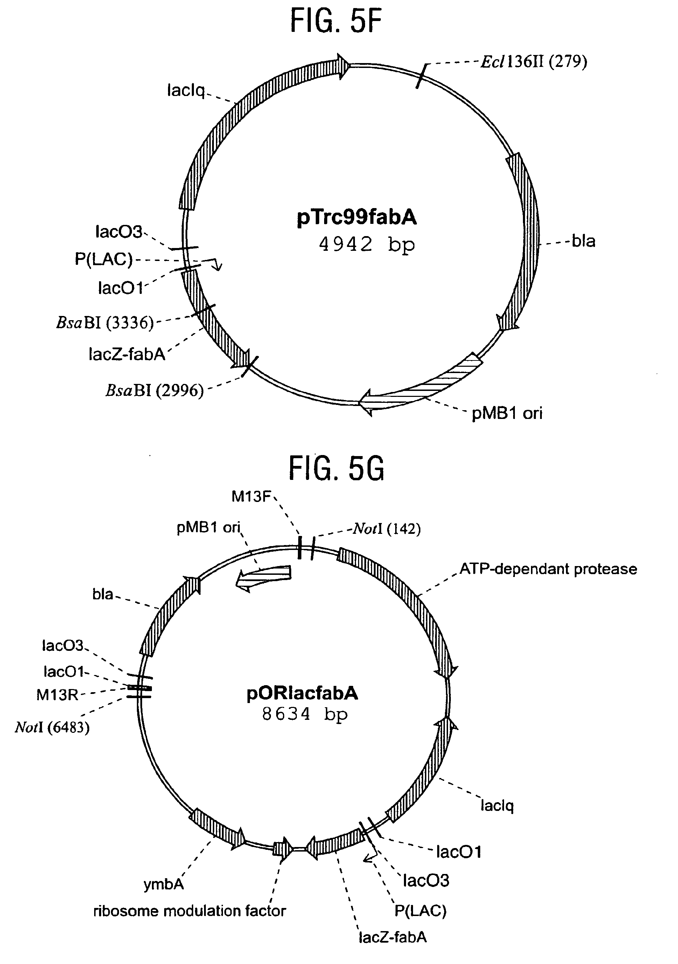

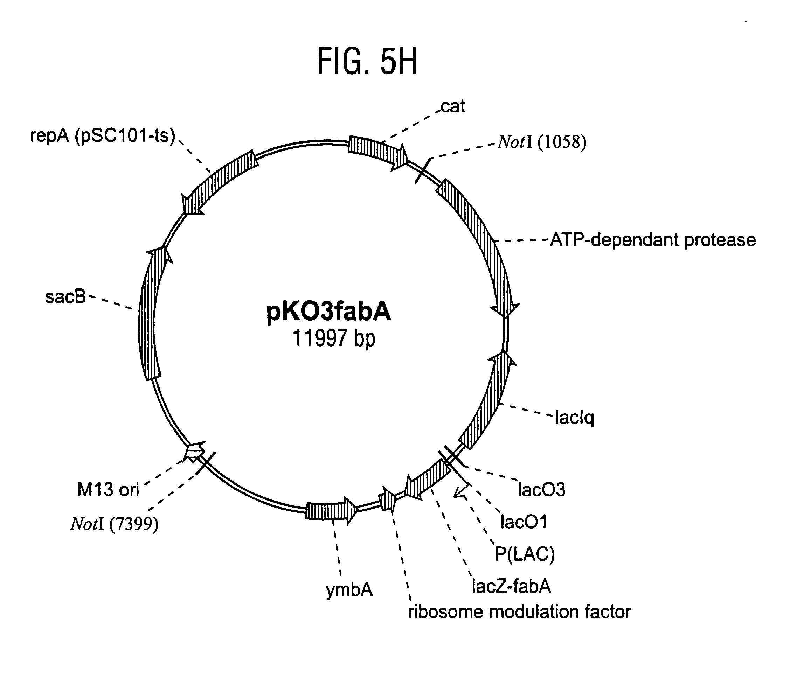

1. Cut pTrc99 (Figure 3A) with *Pfl*MI and blunt ends (with T4 DNA polymerase).
2. Remove the *E.coli lacZ-fabA* expression cassette from placfabA (Figure 5E) as an *EarI-DraIII* fragment and blunt ends.
3. Ligate to create pTrc99fabA (Figure 5F) (orientations of *fabA* and *lacI^{q}* must be opposite).
4. Amplify the *fabA* locus from *Salmonella typhimurium* SL3261 chromosomal DNA by PCR (Taq polymerase) using primers 5STfabA and 3STfabA (these contain *SphI* and *NotI* sites, the PCR product is 4713bp).
5. Clone the PCR product into pCR^{®}2.1-TOPO (Invitrogen), to create pCR2.1St*fabA* (Figure 5C).
6. Cut the *fabA* locus out of pCR2.1 (Figure 5A) as a *NotI* fragment and clone into pORT1 (Figure 5B) cut with *NotI,* to create pORfabA (Figure 5D).
7. Cut *lacZ-fabA* and *lacI^{q}* out of pTrc99fabA (Figure 5F) as an *SmaI-BsaBI* fragment.
8. Cut pORfabA (Figure 5D) with *PmlI-BsaBI* to remove the N-terminal coding region of fabA and ligate the SmaI-BsaBI fragment from pTrc99fabA (Figure 5F) to create pORlacfabA (Figure 5G).
9. Cut out the insert from pORIacfabA (Figure 5G) as a *NotI* fragment and ligate into *NotI*-cut pKO3 to create pKO3fabA (Figure 5H) (insert orientation not important).
10. Use pKOSfabA (Figure 5H) to replace wild-type *fabA* with *lacZ-fabA* in *S. typhimurium* SL3261. The resulting strain is called SL3261 fabA.

### PCR primers used.

*Sphl* and *NotI* sites in bold.
5STfabA(41 nt)
CACCGCGCATGCGGCCGCCCGATGCCAAAGGTATTCGTGTG
3STfabA(41 nt)
TTTGCAGCATGCGGCCGCGTGTGCAGCGTGACGGGTTAGGC

### Example 3: Growth profiles of SL3261 and SL3261dapD

The growth rate of SL3261 in LB was compared to those of SL3261dapD in LB-IPTG.

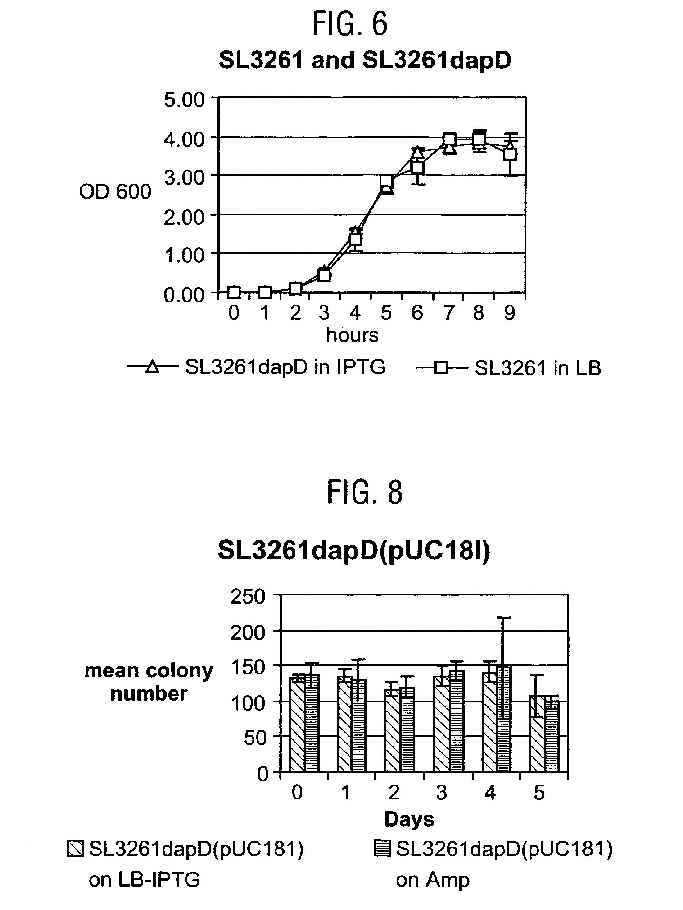

Three SL3261 and SL3261dapD clones were inoculated in 5ml cultures and grown overnight at 37°C in orbital shaker at 200 rpm. The pre-cultures were used to inoculate 50ml cultures to a starting optical density of 0.01 O.D.₆₀₀ and readings were taken hourly (Figure 6).

The growth curves showed no significant difference, indicating that the growth of the ORT strain is not adversely affected by the modifications to *dapD.*

### Example 4: In vitro plasmid maintenance of pUC18I in SL3261dapD

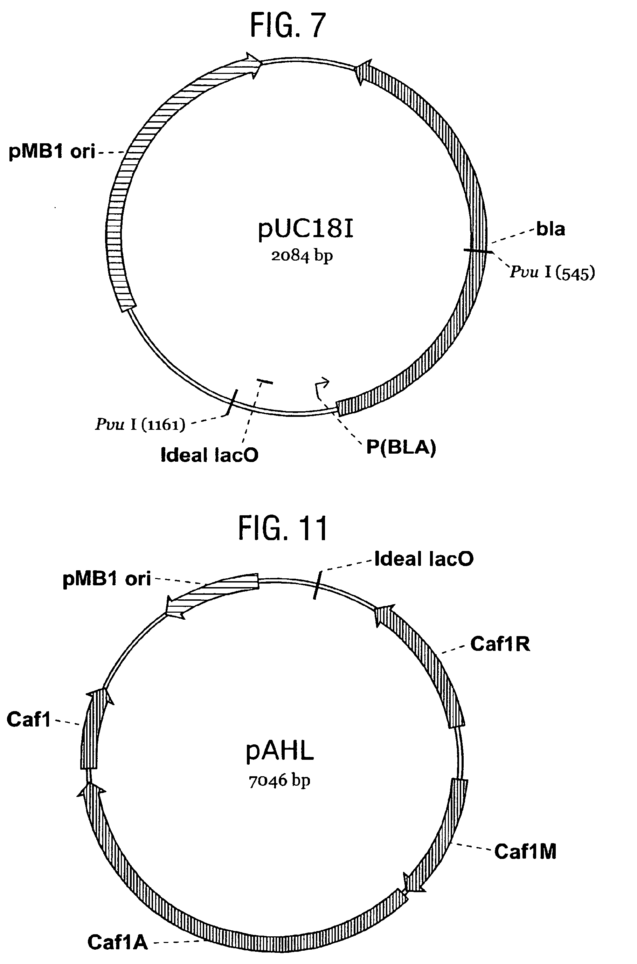

The pUC18I plasmid (Figure 7) has the pMB1 origin of replication and *bla* (ampicillin resistance) from pUC18 as well as an ideal palindromic *lac* operator. The *lac* operator enables ORT, allowing cell growth on a DAP/IPTG free medium.

The plasmid stability of pUC18I in SL3261 dapD was investigated as follows. Three clones were inoculated into 50ml LB ampicillin (25µg/ml) incubated overnight at 37°C in orbital shaker at 200 rpm. The optical density was determined and gave the values for 'day 0', then 50ml fresh LB in the absence of ampicillin were inoculated to 0.001OD₆₀₀, incubated as previously, giving 'day 1'. The process was repeated until 'day 5'.

The cells grew about 11.6 generations per day during five days, so approximately 59 generations throughout the experiment. Number of generations each day = ln(finalOD/initialOD)/ln2.

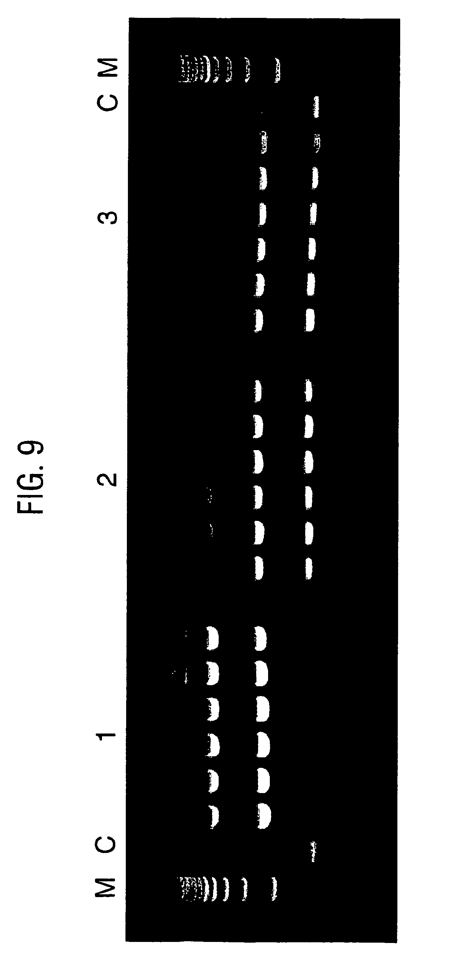

To compare plasmid maintenance, each day 10⁻⁷ ODml were plated out onto permissive and non-permissive media and the clones counted (Figure 8).

Daily 20Dml samples were mini-prepped and the plasmids were examined by agarose gel electrophoresis. AS shown in Figure 9, pUC18I is clearly visible in samples from all time spoints in a highly multimerised state (this is expected as the strain is recombination proficient and the plasmid lacks the correct sites to allow reversion to the monomer state).

### Example 5: In vivo plasmid maintenance of pUC18I in SL3261dapD

### Methods

*S. enterica* serovar Typhimurium strains SL3261, SL3261 (pUC 181) and SL3261 dapD(pUC 18I) were cultured in LB broth supplemented with 1 % (v/v) histidine, 1% (v/v) 'aromix' (4 mg/ml tyrosine, 4 mg/ml phenylalanine, 1 mg/ml *p*-aminobenzoic acid, 1 mg/ml dihydroxybenzoic acid) (to supplement the *aro his* genotype of SL3261). 0.1 mM IPTG was added to SL3261dapD(pUC18I) cultures. Since SL3261(pUC18I) and SL3261dapD(pUC18I) both contained a gene conferring resistance to ampicillin, these strains were cultured in medium also supplemented with 25 µg/ml ampicillin. To produce inocula, *S. enterica* serovar Typhimurium was cultured statically overnight at 37°C, then centrifuged (6000 g, 20 min, 4 °C), washed once in PBS and recentrifuged, and resuspended in PBS to a final cell density of approximately 5 x 10⁹ cfu/ml. Viable bacteria were enumerated on LB agar plates containing IPTG/ampicillin where appropriate.

Groups of 30 female Balb/c mice were inoculated intragastrically with approx. 5x10⁸ cfu/100µl dose of *Salmonella* using a gavage needle. On days 8, 12 and 16 post-inoculation, 10 mice were culled by cervical dislocation and for each mouse the spleen and 6 Peyer's patches (PP) were removed. Schedule used:

| Group | | Day 0 | Day 8 | Day 12 | Day 16 |
|---|---|---|---|---|---|
| | | 12/3/03 | 20/3/03 | 24/3/03 | 28/3/03 |
| A | SL3261- | Inoc 30 | 10 spleen | 10 spleen | 10 spleen |
| | DAPD/pUC18I | | 10 PP | 10 PP | 10 PP |
| B | SL3261/pUC18I | Inoc 30 | 10 spleen | 10 spleen | 10 spleen |
| | | | 10 PP | 10 PP | 10 PP |
| C | SL3261 | Inoc 30 | 10 spleen | 10 spleen | 10 spleen |
| | | | 10 PP | 10 PP | 10 PP |

The spleen or 6 PP were homogenised in 1 ml sterile PBS using 50 µm cell strainers (Becton Dickinson Labware) and suitable dilutions were plated onto suitable agar for enumeration. Specifically, SL3261 was plated onto LB agar only, SL3261(pUC18I) was plated onto LB agar and LB agar containing ampicillin, and SL3261dapD(pUC18I) was plated onto LB agar containing IPTG or LB agar containing ampicillin.

### Results

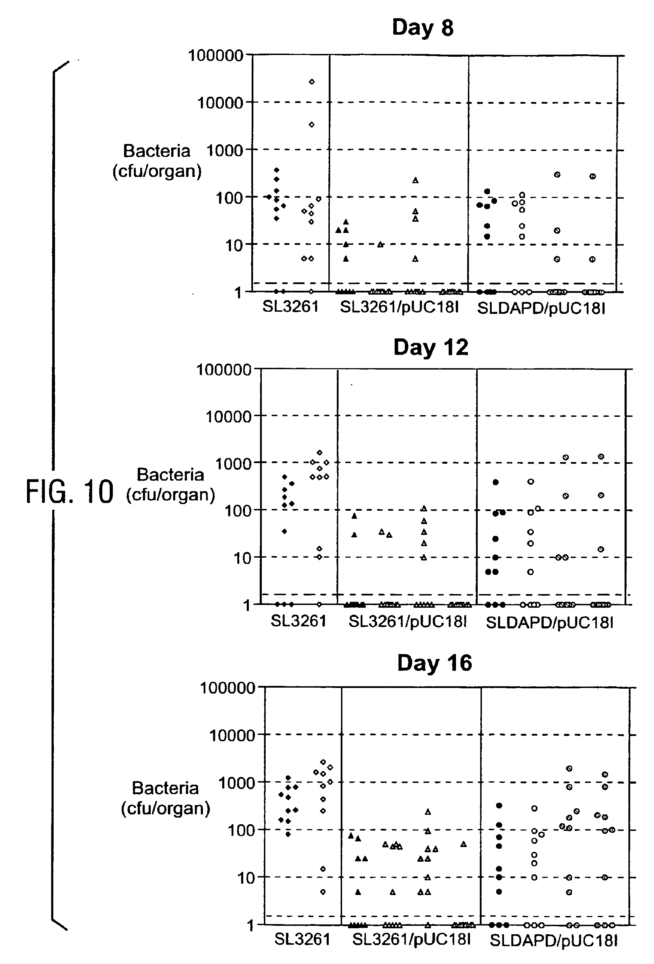

Viable bacteria were detected in the spleens and lungs of all groups of mice inoculated in the experiment (see Figure 10). The following observations were made:
1. SL3261 colonised spleens and PP's as expected.
2. SL3261(pUC18I) was unstable *in vivo.* Significantly lower numbers of SL3261 (pUC 18I) were detected on LB agar plates containing ampicillin compared to LB agar plates, indicating that plasmid pUC18I was lost from the bacteria.
3. SL3261 dapD(pUC18I) was stable *in vivo.* Similar numbers of SL3261dapD(pUC18I)were detected on LB agar plates containing IPTG and LB agar plates containing ampicillin, indicating that plasmid pUC 18I was stabilised.
4. SL3261(pUC18I) and SL3261dapD(pUC18I)(in some cases) were detected as significantly lower numbers in both spleens and PP's compared to SL3261. This may reflect the burden on the *Salmonella* of harbouring the high copy number plasmid pUC18I.

### Conclusions

1. pUC18I was unstable in SL3261 *in vivo,* but stabilised in SL3261dapD.
2. pUC18I reduced the ability of SL3261 and (sometimes) SL3261dapD to colonise spleens and PP's.
3. It is likely that a plasmid with a low copy number, such as pAH34L containing the *lac* operator would be stabilised in SL3261dapD, without the deleterious effects on colonisation.

### Example 6: Growth profiles of SL3261(pAH34L) and SL3261dapD(pAHL)dapD

The pAHL plasmid (Figure 11) has the pSC101 origin of replication, an ideal palindromic *lac* operator as well as the thermo-regulated *caf* operon from *Yersinia pestis.* The *lac* operator enables ORT, allowing cell growth on a DAP/IPTG free medium. Expression at 37°C and not at 28°C of the *caf*1*, caf*1A and *caf*1M genes is controlled by *caf*1R. The pAH34L plasmid is the precursor to pAHL it confers resistance to kanamicin, but lacks the *lac* operator necessary for ORT.

The growth profile of SL3261(pAH34L) in LB-kanamycin was compared to the growth profile of SL3261dapD(pAHL) in LB. Three SL3261(pAH34L) and SL3261dapD(pAHL) clones were inoculated in 5ml cultures and grown over night at 37°C in orbital shaker at 200 rpm. The pre-cultures were used to inoculate 50ml cultures to a starting optical density of 0.01 O.D.₆₀₀ and readings were taken hourly (Figure 12). The growth curves showed no significant difference between these two strains.

### Example 7: In vitro plasmid maintenance of pAHL in SL3261 dapD

The plasmid stability of pAHL in SL3261dapD was investigated as follows. Three clones were inoculated into 50ml LB and incubated over night at 37°C in orbital shaker at 200 rpm. The optical density was determined and gave the values for 'day 0', then 50ml fresh LB were inoculated to 0.001 OD₆₀₀, incubated as previously, giving 'day 1'. The process was repeated until 'day 5'.

The cells grew about 11.6 generations per day during five days (approximately 59 generation throughout the experiment). Number of generations each day = ln(finalOD/initialOD)/ln2.

To demonstrate plasmid maintenance, each day 10⁻⁷ ODml were plated out onto permissive and non-permissive media and the colonies counted. See Figure 13.

Daily 20Dml samples were mini-prepped, the plasmids were examined by agarose gel electrophoresis. The plasmid is clearly visible in samples from all time points (Figure 14). All of these samples prepped from *Salmonella* show a small arte factual band, running below the supercoiled band, which is presumably irreversibly-denatured DNA.

### Summary and conclusions

The strain SL3261dapD allows antibiotic free plasmid selection and maintenance in complex medium (LB) of high and of very low copy number plasmids by ORT. The strain also allows stable *in vivo* maintenance of pUC18I in mice, whereas this plasmid is lost from the parental strain SL3261.

The strain SL3261dapD(pAHL) allows selection and stable maintenance of a plasmid bearing the *caf* operon from *Y.pestis.* This has been show to be a promising vaccine candidate, inducing high levels of immunity against *Y. pestis.* (Titball *et al.,* 1997, Infection and Immunity 65: 1926-1930).

The ORT technology will therefore permit the construction of recombinant strains of *Salmonella* suitable for use as antigen delivery by live vaccine. These strains will not need antibiotic resistance genes for plasmid maintenance. They will also have the advantage of a higher level of antigen expression than would be possible by chromosomal integration of the antigen gene. Furthermore the strain will easily be converted for delivery of different antigens by transformation with appropriate plasmids.

### OTHER EMBODIMENTS

Other embodiments will be evident to those of skill in the art. It should be understood that the foregoing detailed description is provided for clarity only and is merely exemplary. The scope of the present invention is not limited to the above examples, but is defined by the following claims.

## Claims

1. A transformed attenuated bacterial cell for use as a medicament, said cell containing:
a) a plasmid comprising a gene of interest and an operator sequence susceptible to binding by a repressor,
b) a second gene, encoding said repressor, and which is present on a chromosome, and
c) a third gene which is present on the chromosome and is essential for growth and an operator linked in cis to the third gene such that expression of the third gene is susceptible to repression upon binding of a repressor to the operator,
wherein said plasmid is present in said cell in sufficient numbers to titrate said repressor such that said essential gene is expressed, thereby permitting cell growth.

2. A transformed cell according to claim 1, wherein said third gene is not an antibiotic resistance gene.

3. A transformed cell according to claim 1 or claim 2, wherein said transformed cell is an enterobacterium.

4. A transformed cell according to claim 3, wherein said transformed cell is of the genus *Salmonella.*

5. A transformed cell according to claim 4, wherein said transformed cell is of the species *Salmonella typhimurium.*

6. A transformed cell according to claim 4, where said transformed host cell is of the species *Salmonella typhi.*

7. A transformed cell according to any one of claims 4 to 6, wherein said transformed cell has been attenuated by a modification of its *aroA* gene, *aroC* gene, *aroD* gene or *htrA* gene.

8. A transformed cell according to claim 7, wherein said transformed host cell is of the strain *Salmonella typhimurium* SL3261.

9. A transformed cell according to claim 7, wherein said transformed host cell is of the strain *Salmonella typhi* CVD 908-htr.

10. A transformed cell according to any of claims 1 to 9, wherein said third gene contained by the cell encodes an enzyme required for bacterial cell wall synthesis.

11. A transformed cell according to claim 10 wherein said third gene is *dapD.*

12. A transformed cell according to any of claims 1 to 9, wherein said third gene contained by said cell encodes an enzyme required for fatty acid synthesis.

13. A transformed cell according to claim 12 wherein said third gene is *fabA*.

14. A transformed cell according to any of claims 1 to 13, wherein said gene of interest is functionally associated with a bacterial promoter.

15. A transformed cell according to any of claims 1 to 13, wherein said gene of interest is functionally associated with a eukaryotic promoter.

16. A transformed cell according to any of claims I to 15, wherein said gene of interest encodes an antigen.

17. A transformed cell according to any of claims 1 to 15, wherein the transcription product of said gene of interest is an antisense oligonucleotide.

18. Use of a transformed cell as recited in any of claims 1 to 17 in the manufacture of a medicament comprising said transformed cell for gene delivery.

19. Use of a transformed cell as recited in any of claims 1 to 17 in the manufacture of a medicament comprising said transformed cell for immunisation.

20. A composition for use as a medicament comprising a cell as recited in any one of claims 1 to 17 and a pharmaceutically acceptable excipient, diluent or buffer.

## Patentansprüche

1. Transformierte abgeschwächte Bakterienzelle zur Verwendung als Medikament, wobei die Zelle:
a) ein Plasmid, umfassend ein Gen von Interesse und eine Operatorsequenz, welche für das Binden durch einen Repressor empfänglich ist,
b) ein zweites Gen, welches den Repressor kodiert und welches auf einem Chromosom vorliegt, und
c) ein drittes Gen, welches auf dem Chromosom vorliegt und essentiell für das Wachstum ist, und einen Operator, welcher in cis mit dem dritten Gen verbunden ist, sodass die Expression des dritten Gens zur Unterdrückung durch das Binden eines Repressors an den Operator empfänglich ist, enthält,
wobei das Plasmid in der Zelle in ausreichender Anzahl vorliegt, um den Repressor zu titrieren, sodass das essentielle Gen exprimiert wird, wodurch das Zellwachstum ermöglicht wird.

2. Transformierte Zelle nach Anspruch 1, wobei das dritte Gen kein Antibiotika-Resistenzgen ist.

3. Transformierte Zelle nach Anspruch 1 oder Anspruch 2, wobei die transformierte Zelle ein Enterobakterium ist.

4. Transformierte Zelle nach Anspruch 3, wobei die transformierte Zelle von der Gattung *Salmonella* ist.

5. Transformierte Zelle nach Anspruch 4, wobei die transformierte Zelle von der Art *Salmonella typhimurium* ist.

6. Transformierte Zelle nach Anspruch 4, wobei die transformierte Wirtszelle von der Art *Salmonella typhi* ist.

7. Transformierte Zelle nach einem der Ansprüche 4 bis 6, wobei die transformierte Zelle durch eine Modifikation ihres aroA-Gens, aroC-Gens, aroD-Gens oder htrA-Gens abgeschwächt worden ist.

8. Transformierte Zelle nach Anspruch 7, wobei die transformierte Wirtszelle vom Stamm *Salmonella typhimurium* SL3261 ist.

9. Transformierte Zelle nach Anspruch 7, wobei die transformierte Wirtszelle vom Stamm *Salmonella typhi* CVD 908-htr ist.

10. Transformierte Zelle nach einem der Ansprüche 1 bis 9, wobei das dritte von der Zelle beinhaltete Gen ein Enzym kodiert, welches für die Synthese von bakteriellen Zellwänden erforderlich ist.

11. Transformierte Zelle nach Anspruch 10, wobei das dritte Gen *dapD* ist.

12. Transformierte Zelle nach einem der Ansprüche 1 bis 9, wobei das dritte von der Zelle beinhaltete Gen ein Enzym kodiert, welches für die Fettsäuresynthese erforderlich ist.

13. Transformierte Zelle nach Anspruch 12, wobei das dritte Gen *fabA* ist.

14. Transformierte Zelle nach einem der Ansprüche 1 bis 13, wobei das Gen von Interesse funktionell mit einem bakteriellen Promotor verbunden ist.

15. Transformierte Zelle nach einem der Ansprüche 1 bis 13, wobei das Gen von Interesse funktionell mit einem eukaryotischen Promotor verbunden ist.

16. Transformierte Zelle nach einem der Ansprüche 1 bis 15, wobei das Gen von Interesse ein Antigen kodiert.

17. Transformierte Zelle nach einem der Ansprüche 1 bis 15, wobei das Transkriptionsprodukt des Gens von Interesse ein Antisense-Oligonukleotid ist.

18. Verwendung einer transformierten Zelle nach einem der Ansprüche 1 bis 17 in der Herstellung eines Medikaments, umfassend die transformierte Zelle zur Gen-Lieferung.

19. Verwendung einer transformierten Zelle nach einem der Ansprüche 1 bis 17 in der Herstellung eines Medikaments, umfassend die transformierte Zelle zur Immunisierung.

20. Zusammensetzung zur Verwendung als Medikament, umfassend eine Zelle nach einem der Ansprüche 1 bis 17 und ein/einen pharmazeutisch verträgliches/verträglichen Bindemittel, Verdünnungsmittel oder Puffer.

## Revendications

1. Cellule bactérienne atténuée transformée pour utilisation en tant que médicament, ladite cellule contenant :
a) un plasmide comprenant un gène d'intérêt et une séquence opératrice à laquelle un répresseur est susceptible de se lier,
b) un deuxième gène, codant pour ledit répresseur, et qui est présent sur un chromosome, et
c) un troisième gène, qui est présent sur le chromosome et est essentiel pour la croissance, et un opérateur lié en cis au troisième gène de telle sorte que l'expression du troisième gène soit susceptible de subir une répression après liaison d'un répresseur à l'opérateur,
où ledit plasmide est présent dans ladite cellule en des nombres suffisants pour titrer ledit répresseur, de telle sorte que ledit gène essentiel soit exprimé, ce qui permet la croissance cellulaire.

2. Cellule transformée selon la revendication 1, dans laquelle ledit troisième gène n'est pas un gène de résistance aux antibiotiques.

3. Cellule transformée selon la revendication 1 ou 2, où ladite cellule transformée est une entérobactérie.

4. Cellule transformée selon la revendication 3, où ladite cellule transformée est du genre *Salmonella.*

5. Cellule transformée selon la revendication 4, où ladite cellule transformée est de l'espèce *Salmonella typhimurium.*

6. Cellule transformée selon la revendication 4, où ladite cellule hôte transformée est de l'espèce *Salmonella typhi.*

7. Cellule transformée selon l'une quelconque des revendications 4 à 6, où ladite cellule transformée a été atténuée par une modification de son gène *aroA,* de son gène *aroC,* de son gène *aroD* ou de son gène *htrA.*

8. Cellule transformée selon la revendication 7, où ladite cellule hôte transformée est de la souche *Salmonella typhimurium* SL3261.

9. Cellule transformée selon la revendication 7, où ladite cellule hôte transformée est de la souche *Salmonella typhi* CVD 908-htr.

10. Cellule transformée selon l'une quelconque des revendications 1 à 9, dans laquelle ledit troisième gène contenu par la cellule code pour une enzyme exigée pour la synthèse de la paroi cellulaire de bactéries.

11. Cellule transformée selon la revendication 10, dans laquelle ledit troisième gène est *dapD.*

12. Cellule transformée selon l'une quelconque des revendications 1 à 9, dans laquelle ledit troisième gène contenu dans ladite cellule code pour une enzyme nécessaire à la synthèse des acides gras.

13. Cellule transformée selon la revendication 12, dans laquelle ledit troisième gène est *fabA.*

14. Cellule transformée selon l'une quelconque des revendications 1 à 13, dans laquelle ledit gène d'intérêt est fonctionnellement associé à un promoteur bactérien.

15. Cellule transformée selon l'une quelconque des revendications 1 à 13, dans laquelle ledit gène d'intérêt est fonctionnellement associé à un promoteur eucaryote.

16. Cellule transformée selon l'une quelconque des revendications 1 à 15, dans laquelle ledit gène d'intérêt code pour un antigène.

17. Cellule transformée selon l'une quelconque des revendications 1 à 15, dans laquelle le produit de transcription dudit gène d'intérêt est un oligonucléotide antisens.

18. Utilisation d'une cellule transformée selon l'une quelconque des revendications 1 à 17 pour préparer un médicament comprenant ladite cellule transformée, pour livraison de gènes.

19. Utilisation d'une cellule transformée selon l'une quelconque des revendications 1 à 17 pour préparer un médicament comprenant ladite cellule transformée, pour immunisation.

20. Composition pour utilisation en tant que médicament, comprenant une cellule selon l'une quelconque des revendications 1 à 17 et un excipient, un diluant ou un tampon acceptable d'un point de vue pharmaceutique.


### SEQUENCE LISTING

<110> COBRA THERAPEUTICS LIMITED
<120> PLASMID STABILISATION IN VIVO
<130> P033103WO
<140> pct/gb03/02166
   <141> 2003-05-19
<150> GB0211459.3
   <151> 2002-05-18
<160> 6
<170> SeqWin99, version 1.02
<210> 1
   <211> 39
   <212> DNA
   <213> Artificial Sequence
<220>
   <223> Artificial Sequence
<220>
   <223> 5STdapD Primer
<400> 1
   cgaatggcat gcggccgcag ctatttttat caggatttg 39
<210> 2
   <211> 35
   <212> DNA
   <213> Artificial Sequence
<220>
   <223> LST2dapD Primer
<400> 2
   gcgaatgcat agcgggggcg acagcccccc tttgc 35
<210> 3
   <211> 39
   <212> DNA
   <213> Artificial Sequence
<220>
   <223> Artificial Sequence
<220>
   <221> 3STdapD Primer
<400> 3
   tgtggggcat gcggccgcag tggtcgcaca tcgtcggac 39
<210> 4
   <211> 46
   <212> DNA
   <213> Artificial Sequence
<220>
   <223> Artificial Sequence
<220>
   <221> RSTdapD Primer
<400> 4
   gcttttcact cgatgcatct accctttatc gtttggattg agggcc 46
<210> 5
   <211> 41
   <212> DNA
   <213> Artificial Sequence
<220>
   <223> Artificial Sequence
<220>
   <223> 5STfabA Primer
<400> 5
   caccgcgcat gcggccgccc gatgccaaag gtattcgtgt g 41
<210> 6
   <211> 41
   <212> DNA
   <213> Artificial Sequence
<220>
   <223> Artificial Sequence
<220>
   <223> 3STfabA Primer
<400> 6
   tttgcagcat gcggccgcgt gtgcagcgtg acgggttagg c 41